# EUROPEAN PATENT SPECIFICATION

(11) **EP 1 472 351 B1**
(45) Date of publication and mention of the grant of the patent: **20.06.2007**
(21) Application number: 03737422.0
(22) Date of filing: 07.02.2003
(51) Int. Cl.: C12N 15/11, C07K 14/245, C07K 14/285, C07K 14/28, C07K 14/195, C07K 14/21, C07K 14/705, C07K 14/295, C07K 14/32, A61K 39/00, A61K 38/04

(54) **AMINO ACID SEQUENCES CAPABLE OF FACILITATING PENETRATION ACROSS A BIOLOGICAL BARRIER**
AMINOSÄURESEQUENZEN DIE DIE FÄHIGKEIT BESITZEN, DIE DURCHDRINGUNG DURCH EINE BIOLOGISCHE BARRIERE ERLEICHTERN
SEQUENCES D'ACIDES AMINES FACILITANT LA PENETRATION A TRAVERS UNE BARRIERE BIOLOGIQUE

(30) Priority: 07.02.2002 US 355396 P
(43) Date of publication of application: 03.11.2004
(73) Proprietor: YISSUM RESEARCH DEVELOPMENT COMPANY OF THE HEBREW UNIVERSITY OF JERUSALEM, 91390 Jerusalem (IL)
(72) Inventor: BEN-SASSON, Shmuel, A., Jerusalem 96555 (IL); COHEN, Einat, Jerusalem 92548 (IL)
(74) Representative: Crump, Julian Richard John
(86) International application number: PCT/IB2003/000968
(87) International publication number: WO 2003/066859

(56) References cited:
- WO-A-96/05858
- M VAN SCHILFGAARDE ET AL.: "Cloning of genes of nontypeable Haemophilus influentiae involved in penetration between human lung epithelial cells" INFECTION AND IMMUNITY., vol. 68, no. 8, August 2000 (2000-08), pages 4616-4623, XP002258337 AMERICAN SOCIETY FOR MICROBIOLOGY. WASHINGTON., US ISSN: 0019-9567

## Description

Techniques enabling efficient transfer of a substance of interest across a biological barrier are of considerable interest in the field of biotechnology. For example, such techniques may be used for the transport of a variety of different substances across a biological barrier regulated by tight junctions (*i.e.,* the mucosal epithelia, which includes the intestinal and respiratory epithelia and the vascular endothelia, which includes the blood-brain barrier).

The intestinal epithelium represents the major barrier to absorption of orally administered compounds, *e.g.,* drugs and peptides, into the systemic circulation. This barrier is composed of a single layer of columnar epithelial cells (primarily enterocytes, goblet cells, endocrine cells, and paneth cells), which are joined at their apical surfaces by the tight junctions. *See* Madara et al., PHYSIOLOGY OF THE GASTROINTESTINAL TRACT; 2nd Ed., Johnson, ed., Raven Press, New York, pp. 1251-66 (1987).

Compounds that are presented in the intestinal lumen can enter the blood stream through active or facilitative transport, passive transcellular transport, or passive paracellular transport. Active or facilitative transport occurs via cellular carriers, and is limited to transport of low molecular weight degradation products of complex molecules such as proteins and sugars, *e.g.,* amino acids, pentoses, and hexoses. Passive transcellular transport requires partitioning of the molecule through both the apical and basolateral membranes. This process is limited to relatively small hydrophobic compounds. *See* Jackson, PHYSIOLOGY OF THE GASTROINTESTINAL TRACT; 2nd Ed., Johnson, ed., Raven Press, New York, pp. 1597-1621 (1987). Consequently, with the exception of those molecules that are transported by active or facilitative mechanisms, absorption of larger, more hydrophilic molecules is, for the most part, limited to the paracellular pathway. However, the entry of molecules through the paracellular pathway is primarily restricted by the presence of the tight junctions. *See* Gumbiner, Am. J. Physiol., 253:C749-C758 (1987); Madara, J. Clin. Invest., 83:1089-94 (1989).

In transmission electron microscopy, tight junctions appear as an approximately 80 nm long region at the boundary of neighboring cells in which the plasma membranes of adjacent cells are brought into close opposition. *See* Farquhar, et al., J. Cell Biol., 17:375-412 (1963). This structure circumscribes epithelial cells immediately below the brush border (apical domain), thereby forming a seal between epithelial cells and their neighbors. *See* Pappenheimer, et al., J. Membrane Biol., 102:2125-2136 (1986); Claude et al., J. Cell Biol., 58:390-400 (1973); and Bakker, et al., J. Membrane Biol., 98:1209-1221 (1985). The tight junctions function to define apical and basal membrane polarity by limiting the exchange of membrane lipids and proteins between the two and to regulate the paracellular transport of water, solutes, and immune cells. *See* Heiskala, et al., Traffic, 2:92-98 (2001).

Considerable attention has been directed to finding ways to increase paracellular transport by "loosening" tight junctions. One approach to overcoming the restriction to paracellular transport is to co-administer, in a mixture, biologically active ingredients with absorption enhancing agents. Generally, intestinal/respiratory absorption enhancers include, but are not limited to, calcium chelators, such as citrate and ethylenediamine tetraacetic acid (EDTA); surfactants, such as sodium dodecyl sulfate, bile salts, palmitoylcarnitine, and sodium salts of fatty acids; or toxins, such as zonula occludens toxin ("ZOT"). ZOT functions by increasing the bioavailability of oral insulin in diabetic animals. *See* Fasano and Uzzau, J. Clin. Invest., 99:1158-64 (1997). EDTA, which is known to disrupt tight junctions by chelating calcium, enhances the efficiency of gene transfer into the airway respiratory epithelium in patients with cystic fibrosis. *See* Wang, et al., Am. J. Respir. Cell Mol. Biol., 22:129-138 (2000). However, one drawback to all of these methods is that they facilitate the indiscriminate penetration of any nearby molecule that happens to be in the gastrointestinal or airway lumen. In addition, each of these intestinal/respiratory absorption enhancers has properties that limit their general usefulness as a means to promote absorption of various molecules across a biological barrier.

Moreover, with the use of surfactants, the potential lytic nature of these agents raises concerns regarding safety. Specifically, the intestinal and respiratory epithelia provides a barrier to the entry of toxins, bacteria and viruses from the hostile exterior. Hence, the possibility of exfoliation of the epithelium using surfactants, as well as the potential complications arising from increased epithelial repair, raise safety concerns about the use of surfactants as intestinal/respiratory absorption enhancers.

When calcium chelators are used as intestinal/respiratory absorption enhancers, Ca⁺² depletion does not act directly on the tight junction, but, rather, induces global changes in the cells, including disruption of actin filaments, disruption of adherent junctions, diminished cell adhesion, and activation of protein kinases. *See* Citi, J. Cell Biol., 117:169-178 (1992). Moreover, as typical calcium chelators only have access to the mucosal surface, and luminal Ca⁺² concentration may vary, sufficient amounts of chelators generally cannot be administered to lower Ca ⁺² levels to induce the opening of tight junctions in a rapid, reversible, and reproducible manner.

Additionally, some toxins such as *Clostridium difficile* toxin A and B, appear to irreversibly increase paracellular permeability and are thus, associated with destruction of the tight junction complex. *See* Hecht, et al., J. Clin. Invest., 82:1516-24 (1988); Fiorentini and Thelestam, Toxicon, 29:543-67 (1991). Other toxins such as *Vibrio cholerae* zonula occludens toxin (ZOT) modulate the structure of intercellular tight junctions. As a result, the intestinal mucosa becomes more permeable. *See* Fasano, et al., Proc. Nat. Acad Sci., USA, 8:5242-46 (1991); U.S. Patent No. 5,827,534. However, this also results in diarrhea.

Other penetrating structures are described in WO-A-9605858 (Washington & Stanford Universities) and Inf. Immun. 68(8), pages 4416-4423.

Thus, a need remains for an efficient, specific, non-invasive, low-risk means to target various biological barriers for the delivery of biologically active molecules such as polypeptides, drugs and other therapeutic agents.

The present invention therefore provides a penetrating module comprising a penetrating peptide and an effector that is coupled or fused to said penetrating peptide, which penetrating peptide capable of translocating across a biological barrier, as claimed in claim 1 below. The invention also relates to an *ex vivo* method of using such a penetrating peptide to translocate an effector across a biological barrier and to methods of producing such a penetrating module.

Said penetrating peptide has an amino acid sequence selected from any one of SEQ ID NOS: 1-15 and 24-29. In various embodiments, the penetrating peptide can be less than thirty (30), less than twenty-five (25), or less than twenty (20) amino acids in length.

As used herein, a "penetrating peptide" is any peptide that facilitates the translocation of a substance across a biological barrier. Examples of biological barriers include, but are not limited to, tight junctions and the plasma membrane. Moreover, those skilled in the art will recognize that translocation may occur across a biological barrier in a tissue such as epithelial cells or endothelial cells.

Said penetrating peptide is fused, coupled or attached to an effector. The "effector" can be any suitable molecule, including, but not limited to DNA, RNA, a protein, a peptide, or a pharmaceutically active agent, such as, for example, a hormone, a growth factor, a neurotrophic factor, a bioactive peptide, heparin, a toxin, an antibiotic, an antipathogenic agent, an antigen, an antibody, an antibody fragment, an immunomodulator, and an enzyme; or a therapeutic agent. Bioactive peptides include, for example, insulin, growth hormone, a gonadotropin, a growth factor, erythropoietin, granulocyte/monocyte colony stimulating factor (GM-CSF), αMSH, enkephalin, dalargin, kyotorphin, EPO, bFGF, hirulog, a lutenizing hormone releasing hormone (LHRH) analog, and neurotrophic factors. Pharmaceutically active agents include, for example, an anticoagulant, a toxin, an antibiotic, an antipathogenic agent, an antigen, an antibody fragment, a vitamin, an immunomodulator, an enzyme, an antineoplastic agent, heparin, methotraxate, and a therapeutic agent.

As used herein, the terms "fusion" or "fused" or "coupled" or "attached" are meant to include all such specific interactions that result in two or more molecules showing a preference for one another relative to some third molecule including any type of interaction enabling a physical association between an effector or a molecular vessel and a penetrating peptide. This includes, but is not limited to, processes such as covalent, ionic, hydrophobic, and hydrogen bonding, but does not include non-specific associations such as solvent preferences. The association must be sufficiently strong so that the effector does not disassociate before or during penetration of the biological barrier. Fusion may be achieved using any chemical, biochemical, enzymatic or genetic coupling method known to those skilled in the art. The effector of interest is preferably coupled to the C-terminal end of the penetrating peptide.

In other embodiments, the penetrating peptide could also be a complex consisting of the penetrating peptide attached to a "molecular vessel" in which an effector is enclosed. Suitable effectors include, but are not limited to, any of the suitable bioactive peptides or pharmaceutically active agents described herein. A molecular vessel could be a soluble receptor, or part of a soluble receptor (*e.g.,* a "minireceptor", as described in Kristensens, et al., J. Biol. Chem., 274(52):37351-56 (1999)). A molecular vessel can also be a binding protein. The molecular vessel will serve as a high affinity binding pocket for the delivery of the intact effector, such as a hormone, a growth factor, or any other effector. One example of a molecular vessel is a soluble insulin receptor, or the ligand-binding domain of the insulin receptor (*e.g.,* the above-mentioned "minireceptor'), to bind and deliver insulin as an effector. Another example for the use of a molecular vessel to deliver non-permeable effectors, is the use of Intrinsic factor, attached to the penetrating peptide, in order to enable the delivery of vitamin B12 as an effector. In a way, the molecular vessel serves as a "Trojan horse" to translocate the otherwise impermeable effector across a biological barrier. The "effector" can be any suitable molecule as defined above.

The peptides described herein may serve as the basis for the design of therapeutic drug-containing micro particles/droplets. For example, penetrating peptides are attached to fatty moieties and incorporated at the interface of a hydrophobic vesicle which contains the desired pharmaceutical composition. This can be achieved via amidation of the free amino group of extra lysine(s), added at the C-terminus of the penetrating peptide, using long fatty acids such as stearoyl, palmitoyl or myristoyl.

Another embodiment of the invention involves the use of the penetrating module of the present invention in the manufacture of a vaccine for oral vaccination by administration to a subject. Said penetrating peptide may be attached to a desirable antigenic protein. In one embodiment, the invention involves an *ex vivo* method of translocating an effector across a biological barrier by coupling the effector to a penetrating peptide module that can be introduced to the biological barrier.

As used herein, the term "biological barrier" is meant to include biological membranes such as the plasma membrane as well as any biological structures sealed by tight junctions (or occluding junctions) such as the mucosal epithelia, including, but not limited to, the intestinal or respiratory epithelia or the vascular endothelia, including, but not limited to, the blood-brain barrier.

In still further embodiments, the invention includes a pharmaceutical composition containing a therapeutically or prophylactically effective amount of a penetrating module according to the invention and a pharmaceutically acceptable carrier.

Preferred "pharmaceutical compositions" include enteric coated tablets and gelatin capsules comprising the active ingredient together with a) diluents, *e.g.,* lactose, dextrose, sucrose, mannitol, sorbitol, cellulose and/or glycine; b) protease inhibitors such as Aprotinin or trasylol; c) lubricants, *e.g.,* silica, talcum, stearic acid, its magnesium or calcium salt, poloxamer and/or polyethyleneglycol; for tablets also d) binders, *e.g.,* magnesium aluminum silicate, starch paste, gelatin, tragacanth, methylcellulose, sodium carboxymethylcellulose and/or polyvinylpyrrolidone; e) ionic surface active agents such as bile salts, if desired f) disintegrants, *e.g.,* starches, agar, alginic acid or its sodium salt, or effervescent mixtures; and/or g) absorbents, colorants, flavors and sweeteners. Injectable compositions are preferably aqueous isotonic solutions or suspensions, and suppositories are advantageously prepared from fatty emulsions or suspensions. The compositions may be sterilized and/or contain adjuvants, such as preserving, reducing agents *e.g.,* NAC (N-Acetyl-L-Cysteine), stabilizing, wetting or emulsifying agents, solution promoters, salts for regulating the osmotic pressure and/or buffers. In addition, they may also contain other therapeutically valuable substances. The compositions are prepared according to conventional mixing, granulating or coating methods, respectively, and contain about 0.01 to 75%, preferably about 0.1 to 10%, of the active ingredient.

These compositions may further contain a mixture of at least two substances selected from the group consisting of a non-ionic detergent, an ionic detergent, a protease inhibitor, and a reducing agent. For example, the non-ionic detergent may be a poloxamer; the poloxamer may be pluronic F-68; the ionic detergent may be a bile salt; and the bile salt may be Taurodeoxychilate; the protease inhibitor may be selected from the group consisting of aprotonir and soy bean trypsin inhibitor, and/or the reducing agent may be NAC.

The invention also provides a method for producing a penetrating module by coupling an effector to a penetrating peptide. For example, said penetrating module can be produced by transfecting a production cell with a vector that has a nucleic acid molecule of a fusion protein encoding the penetrating peptide and an effector operably linked to an expression control sequence; culturing the production cell under conditions that permit production of a fusion protein that includes the penetrating peptide and an effector peptide; and isolating the fusion protein. For example, the penetrating module can also be produced by using solid-phase peptides synthesis methods, as is well known in the art. Penetrating modules that are produced by the methods for producing a penetrating peptide of the invention can also be further chemically modified. For example, one or more polyethylene glycol (PEG) residues can be attached to the penetrating peptides of the invention.

In another embodiment, the effector may be coupled to the penetration peptide by a covalent or a non-covalent bond. For example, the covalent bond may be a peptide bond or the covalent bond may be achieved by a homo- or a hetero-functional bridging reagent. The bridging reagent may be a succinimidyl-(N-maleimidomethyl) cyclohexane-1-carboxylate (SMCC)-type reagent. The covalent bond may also be achieved by using a peptide linker. In some embodiments, the peptide linker may have an amino acid sequence of SEQ ID NO: 16 or 17 and may be cleaved by an enzyme. In some embodiments, the enzyme may be conditionally activated under a certain physiological state, and the released factor may favorably influence that physiological state. In other embodiments, the non-covalent bond may be achieved by an attachment of a hydrophobic moiety to the penetrating peptide, such that the hydrophobic moiety enables the penetrating peptide to be incorporated at the interface of a hydrophobic vesicle in which the effector is contained. In other embodiments, the non-covalent bond may be the result of a biotin-avidin or biotin-streptavidin interaction.

In various other embodiments, the penetrating peptides are derived from a pathogenic or non-pathogenic bacteria, characterized by the ability to penetrate biological barriers *in vivo*. In some embodiments, the penetrating peptides are derived from integral membrane proteins of pathogenic or non-pathogenic bacteria. In other embodiments, the penetrating peptides are derived from extracellular proteins released by pathogenic or non-pathogenic bacteria. In other embodiments, the penetrating peptide is derived from a bacterial toxin. In still other embodiments, the penetrating peptides are derived from human neurokinin receptors, characterized by the ability to penetrate biological barriers in *vivo,* or are derived from viral proteins.

In still further embodiments the concept of a conditionally-activated effector is designed to allow selective release and activation of proteins under conditions where and at sites in which their activity is beneficial.

In this embodiment, the beneficial protein (the effector) is coupled to the penetrating peptide through a cleavable linker peptide. The cleavage site is endogenously recognized and cleaved by an enzyme of the cascade targeted by the effector. The released effector may act as an inhibitory protein or an activator of desired processes.

Finally, another embodiment of the invention involves the use of a penetrating module in accordance with the invention in the manufacture of a medicament for treating or preventing a disease or pathological condition by administration to a subject in which such treatment or prevention is desired. For example, the disease or condition to be treated may include but are not limited to endocrine disorders, including diabetes, infertility, hormone deficiencies and osteoporosis; neurodegenerative disorders, including Alzheimer's disease, Parkinson's disease, and Huntington's disease; cardiovascular disorders, including atherosclerosis, hyper- and hypocoagulable states, coronary disease, and cerebrovascular events; metabolic disorders, including obesity and vitamin deficiencies; haematological disorders; and neoplastic disease.

The details of one or more embodiments of the invention have been set forth in the accompanying description below. Although any methods and materials similar or equivalent to those described herein can be used in the practice or testing of the present invention, the preferred methods and materials are now described. Other features, objects, and advantages of the invention will be apparent from the description and from the claims. In the specification and the appended claims, the singular forms include plural referents unless the context clearly dictates otherwise. Unless defined otherwise, all technical and scientific terms used herein have the same meaning as commonly understood by one of ordinary skill in the art to which this invention belongs.

The advantages of the use of small peptide carriers include high quality and purity, low immunogenicity and the potential for highly efficient delivery to biological barriers in an organism. Accordingly, peptide carriers have the potential to improve upon conventional transporters such as liposomes or viruses for the efficient delivery of many macromolecules. The present invention employs a short peptide motif to create a penetrating module specifically to transport macromolecules across biological barriers sealed by tight junctions.

The penetration module of the present invention specifically targets various tissues, especially epithelial and endothelial ones, for the delivery of drugs and other therapeutic agents across a biological barrier. Existing transport systems in the art are too limited to be of general application because they are inefficient, alter the biological properties of the active substance, kill the target cell, irreversibly destroy the biological barrier and/or pose too high of a risk to be used in human subjects.

The present invention uses conserved peptide sequences from various proteins involved in paracytosis to create a penetrating module capable of crossing biological barriers. For example, a peptide encoded by or derived from ORF HI0638 of *Haemophilus influenzae* facilitates penetration of this bacterium between human lung epithelial cells without compromising the epithelial barrier. The peptide sequence encoded by ORF HI0638 is conserved in common pathogenic bacteria or symbiotic including, for example, *Haemophilus influenzae, Pasteurella mullocida, Escherichia coli, Vibrio cholerae, Buchnera aphidicola, Pseudomonas aeruginosa,* and *Xylella fastidiosa.* A peptide homologous to the N-terminal sequence of HI0638 is also found in other bacteria including, for example, *Rhizobium loti, Chlamydia pneumoniae,* NprB from *Bacillus subtilis,* and pilins from *Kingella dentrificans* and *Eikenella corrodens.*

Furthermore, a similar peptide sequence is also conserved in proteins of eukaryotic origin such as the neurokinin receptor family proteins, including the human NK-1 and NK-2 receptors. It is known that the neurokinin receptor family is involved in the control of intercellular permeability including plasma extravasation and oedema formation. Extravasation, the leakage and spread of blood or fluid from vessels into the surrounding tissues, often follows inflammatory processes involved in tissue injury, allergy, bums and inflammation. In particular, when NK-1 receptors on blood vessels are activated skin inflammation occurs due to an increase in vascular permeability. *See* Inoue, et al., Inflamm. Res., 45:316-323 (1996). The neurokinin NK-1 receptor also mediates dural and extracranial plasma protein extravasation, thereby implicating the NK-1 receptor in the pathophysiology of migraine headache. *See* O'Shaughnessy and Connor, Euro. J. of Pharm., 236:319-321 (1993).

The sequences of example penetrating peptides for use in the present invention are shown in Table 1.

**TABLE 1**

| **Peptide/Organism** | **Sequence** | **SEQ ID NO** |
|---|---|---|
| Peptide 1: from ORF HI0638 *Haemophilus influenzae* | NYHDIVLALAGVCQSAKLVHQLA | (SEQ ID NO:1) |
| Peptide 2: from PM1850 *Pasteurella multocida* | NYYDITLALAGVCQAAXLVQQFA | (SEQ ID NO:2) |
| Peptide 3: from YCFC *Escherichia coli* | NYYDITLALAGICQSARLVQQLA | (SEQ ID NO:3) |
| Peptide 4: from VC1127 *Vibrio cholerae* | AIYDRTIAFAGICQAVALVQQVA | (SEQ ID NO:4) |
| Peptide 5: from BU262 *Buchnera aphidicola* | KIHLITLSLAGICQSAHLVQQLA | (SEQ ID NO:5) |
| Peptide 6: from PA2627 *Pseudomonas aeruginosa* | DPRQQLIALGAVFESAALVDKLA | (SEQ ID NO:6) |
| Peptide 7: from XF1439 *Xylella fastidiosa* | LIDNRVLALAGVVQALQQVRQIA | (SEQ ID NO:7) |
| Peptide 8: from MLR0187 *Rhizobium loti* | NLPPIVLAVIGICAAVFLLQQYV | (SEQ ID NO:8) |
| Peptide 9: from Human NK-2 Receptor | NYFIVNLALADLCMAAFNAAFNF | (SEQ ID NO:9) |
| Peptide 10: from CPN0710/C *Chlamydia pneumoniae* | TAFDFNKMLDGVCTYVKGVQQYL | (SEQ ID NO:10) |
| Peptide 11: from MLR4119 *Rhizobium loti* | RAILIPLALAGLCQVARAGDISS | (SEQ ID NO:11) |
| Peptide 12: from NprB *Bacillus subtilis* | MRNLTKTSLLLAGLCTAAQMVFVTH | (SEQ ID NO:12) |
| Peptide 13: from Pilin *Kingella dentrificans* | IELMIVIAIIGILAAIALPAYQEYV | (SEQ ID NO:13) |
| Peptide 14: from Pilin *Eikenella corrodens* | IELMIVIAIIGILAAIALPAYQDYV | (SEQ ID NO: 14) |
| Peptide 15: from zonula occludens toxin (ZOT) | ASFGFCIGRLCVQDGF | (SEQ ID NO:15) |
| Peptide 29: from Human NK-1 Receptor | NYFLVNLAFAEASMAAFNTVVNF | (SEQ ID NO:24) |
| Peptide 30: from YCFC *Escherichia coli* | MNYYDITLALAGICQSARLVQQLA | (SEQ ID NO:25) |
| Peptide 31: from YCFC *Escherichia coli* | MYYDITLALAGICQSARLVQQLA | (SEQ ID NO:26) |
| Peptide 32: from YCFC *Escherichia coli* | MYDTTLALAGICQSARLVQQLA | (SEQ ID NO:27) |
| Peptide 33: from NprB *Bacillus subtilis* | MRNLTRTSLLLAGLCTAAQMVFV | (SEQ ID NO:28) |
| Peptide 34: from ORF HI0638 *Haemophilus influenzae* | NYHDIVLALAGVCQSARLVHQLA | (SEQ ID NO:29) |

Alternatively, the penetrating module of the instant invention may include a peptide containing at least 12 contiguous amino acids of any of the peptides defined by SEQ ID NOS:1-15 and 24-29.

The penetration module of the present invention exhibits efficient, non-invasive delivery of an unaltered biologically active substance. For example, the penetrating modules of the invention can be used in the treatment of diabetes. Insulin levels in the blood stream must be tightly regulated. The penetrating modules of the invention can be used to deliver insulin across the mucosal epithelia at high yield. Alternative non-invasive insulin delivery methods, previously known in the art, have typical yields of 1-5% and cause intolerable fluctuations in the amount of insulin absorbed.

In addition, these penetrating modules can be used to treat conditions resulting from atherosclerosis and the formation of thrombi and emboli such as myocardial infarction and cerebrovascular accidents. Specifically, the penetrating modules can be used to deliver heparin across the mucosal epithelia.

The penetrating modules of the invention also can be used to treat female fertility problems. For example, the penetrating modules can be used to transport follicle stimulating hormone ("FSH") across the mucosal epithelia.

Previously, the delivery of effectors (*e.g.,* the delivery of insulin or heparin to the blood stream) required invasive techniques such as intravenous or intramuscular injections. One advantage of the penetrating modules is that they can deliver effectors across biological barriers through non-invasive administration, including, for example oral, bucal, inhalation, insufflation, transdermal, or depository. In addition, a further advantage of the penetrating modules of the invention is that they can cross the blood-brain barrier, thereby delivering effectors to the CNS.

The peptides described herein may serve as the basis for the design of therapeutic "cargos", namely the coupling of the carriers ("penetrating peptide") with one or more therapeutic agents ("effectors"). A covalent or noncovalent bond can be used to couple a penetrating peptide to one or more effectors or molecular vessels. Peptide linkers that are cleaved by endogenous peptidases can be used to couple penetrating peptides and effectors or molecular vessels. One example of a detachable peptide linker is the amino acid sequence IEGR (SEQ ID NO:16) that can be cleaved in the bloodstream by factor Xa (*see, e.g.,* Karlheinz, P., et al., Circulation 101:1158-1164 (2000)).

Peptide linkers are short sequences appearing between the amino acid sequences of the penetrating peptide and the effector. These peptide linkers are cleaved by endogenous peptidases or other enzymes, thereby dissociating the coupling of penetrating peptide to the effector. Such peptide linkers can be utilized for the creation of conditionally activated effectors. Conditionally activated effectors are coupled to penetrating peptides by peptide linkers that are preferentially cleaved during specific physiological processes. Once cleaved, the effector is released to modulate the process - for instance, inhibition of a signal cascade. Preferential cleavage is achieved by using linker sequences cleaved by proteases that are physiologically activated by the triggered cascade. Thus, the conditionally activated effector serves as a "negative feedback" mechanism that inhibits the physiologic process which activated it.

One example of a conditionally activated effector is a thrombolysis activator, *e.g.*, tPA (tissue plasminogen activator). By using the above mentioned linker (*i.e.,* SEQ ID NO. 16) for cleavage by factor Xa, tPA is preferentially released in sites of active coagulation. This way excessive coagulation cascades can be modulated to reduce pathologic thrombosis due to the local release oftPA. Alternatively, other coagulation inhibitors may be used in place of tPA, *e.g.,* hirudin, hirolog, protein C, protein C activator or protein S.

Another example of conditionally activated effectors is a complement cascade inhibitor, *e.g.,* Clq inhibitor. In this example a peptide linker is incorporated that is specifically cleaved by elements of the complement cascade, *e.g.,* C3a. Thus, excessive complement activation is inhibited, thereby attenuating the extent of pathologic inflammatory processes.

By way of non-limiting example, conditionally activated effectors according to the invention may be used, *e.g.,* in the inhibition of excessive blood clotting, in the inhibition of pathological inflammatory processes, in the inhibition of high blood pressure and congestive heart failure by angiotensin converting enzyme (ACE)-dependent release of an appropriate effector such as brain-derived natriuric peptide (BNP), or in the inhibition of excessive extracellular-matrix degradation, such as the degradation that occurs during inflammatory processes or tumor invasion, by matrix metaloproteinases (MPP)-dependent release of an effector such as tissue inhibitor of metaloproteinases (TIMP).

Alternatively, the penetrating peptide can be attached to a linker to which imaging compounds can be covalently attached, for example through free amino groups of lysine residues. Such a linker includes, but is not limited to, the amino acid sequence GGKGGK (SEQ ID NO:17).

A penetrating peptide is a peptide that facilitates the passage, translocation or penetration of a substance across a biological barrier, particularly between cells "sealed" by tight junctions. Translocation may be detected by any method known to those skilled in the art, including using radioactive tagging and/or fluorescent probes incorporated into a penetrating module in conjunction with a paracytosis assay as described in, for example, Schilfgaarde, et al., Infect. and Immun., 68(8):4616-23 (2000). Generally, a paracytosis assay is performed by: a) incubating a cell layer with a penetrating peptide or module; b) making cross sections of the cell layers; and c) detecting the presence of the peptides or peptide modules. The detection step may be carried out by incubating the fixed cell sections with labeled antibodies directed to the peptide, followed by detection of an immunological reaction between the peptide and the labeled antibody. Alternatively, the peptide may be labeled using a radioactive label, or a fluorescent label, or a dye in order to directly detect the presence of the peptide. Further, a bioassay can be used to monitor the peptide translocation. For example, using a bioactive peptide such as insulin, attached to a penetrating module, the drop in blood glucose level can be measured.

As used herein, the term "effector" refers to any molecule or compound of, for example, biological, therapeutic, pharmaceutical, diagnostic, tracing, or food processing interest. It may consist of nucleic acids (ribonucleic acid, deoxyribonucleic acid) from various origins, and particularly of human, viral, animal, eukaryotic or prokaryotic, plant, synthetic origin, etc. A nucleic acid of interest may be of a variety of sizes, ranging from, for example, a simple trace nucleotide to a genome fragment, or an entire genome. It may be a viral genome or a plasmid. Alternatively, the effector of interest may also be a protein, such as, for example, an enzyme, a hormone, a cytokine, an apolipoprotein, a growth factor, a bioactive peptide, an antigen, or an antibody, etc. Furthermore, the effector may be a pharmaceutically active agent, such as, for example, a toxin, a therapeutic agent, or an antipathogenic agent, such as an antibiotic, an antiviral, an antifungal, or an anti-parasitic agent. The effector can also be a physiologically active molecule like a vitamin. The effector of interest may itself be directly active or may be activated *in situ* by the peptide, by the molecular vessel, by a distinct substance, or by environmental conditions.

The terms "pharmaceutically active agent" and "therapeutic agent" are used herein to refer to a chemical material or compound which, when administered to an organism, induces a detectable pharmacologic and/or physiologic effect.

The penetrating modules according to the present invention are characterized by the fact that their penetration capacity is virtually independent of the nature of the effector or molecular vessel that is coupled to it.

Also included in the invention are methods of producing the penetrating modules described herein. For example, a penetrating module of the invention can be produced by standard recombinant DNA techniques known in the art. By way of non-limiting example, DNA fragments coding for the different polypeptide sequences can be ligated together in-frame in accordance with conventional techniques, *e.g.*, by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers.

Alternatively, PCR amplification of gene fragments can be carried out using anchor primers that give rise to complementary overhangs between two consecutive gene fragments that can subsequently be annealed and reamplified to generate a chimeric gene sequence (*see, e.g.,* Ausubel, et al. (eds.) CURRENT PROTOCOLS IN MOLECULAR BIOLOGY, John Wiley & Sons, 1992). Moreover, many expression vectors are commercially available that already encode a fusion moiety (*e.g.*, a GST polypeptide). A penetrating peptide-encoding nucleic acid can be cloned into such an expression vector such that the fusion moiety is linked in-frame to the penetrating peptide.

As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e.g.,* bacterial vectors having a bacterial origin of replication and episomal mammalian vectors). Other vectors (*e.g.*, non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e.g.*, replication defective retroviruses, adenoviruses and adeno-associated viruses), which serve equivalent functions.

Recombinant expression vectors comprise a nucleic acid in a form suitable for expression of the nucleic acid in a host cell, which means that the recombinant expression vectors include one or more regulatory sequences, selected on the basis of the host cells to be used for expression, that is operatively-linked to the nucleic acid sequence to be expressed. Within a recombinant expression vector, "operably-linked" is intended to mean that the nucleotide sequence of interest is linked to the regulatory sequence(s) in a manner that allows for expression of the nucleotide sequence (*e.g.,* in an *in vitro* transcription/translation system or in a host cell when the vector is introduced into the host cell).

The term "regulatory sequence" is intended to include promoters, enhancers and other expression control elements (*e.g.,* polyadenylation signals). Such regulatory sequences are described, for example, in Goeddel, GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (*e.g.*, tissue-specific regulatory sequences). It will be appreciated by those skilled in the art that the design of the expression vector can depend on such factors as the choice of the host cell to be transformed, the level of expression of protein desired, etc. Expression vectors can be introduced into host cells to thereby produce proteins or peptides, including fusion proteins or peptides, encoded by nucleic acids as described herein (*e.g.*, penetrating peptides or modules).

Recombinant expression vectors can be designed for expression of penetrating peptides or modules in prokaryotic or eukaryotic cells. For example, penetrating peptides or modules can be expressed in bacterial cells such as *Escherichia coli,* insect cells (using baculovirus expression vectors), yeast cells or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated *in vitro,* for example using T7 promoter regulatory sequences and T7 polymerase.

Expression of proteins in prokaryotes is most often carried out in *Escherichia coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: (*i*) to increase expression of recombinant protein; (*ii*) to increase the solubility of the recombinant protein; and (*iii*) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson, 1988. Gene 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion *E. coli* expression vectors include pTrc (Amrann et al., (1988) Gene 69:301-315) and pET 11d (Studier et al., GENE EXPRESSION TECHNOLOGY: METHODS IN ENZYMOLOGY 185, Academic Press, San Diego, Calif. (1990) 60-89).

One strategy to maximize recombinant protein expression in *E. coli* is to express the protein in a host bacteria with an impaired capacity to proteolytically cleave the recombinant protein. *See, e.g.,* Gottesman, GENE EXPRESSION TECHNOLOGY: METHODS IN ENZYMOLOGY 185, Academic Press, San Diego, Calif. (1990) 119-128. Another strategy is to alter the nucleic acid sequence of the nucleic acid to be inserted into an expression vector so that the individual codons for each amino acid are those preferentially utilized in *E. coli* (*see, e.g.,* Wada, et al., 1992. Nucl. Acids Res. 20: 2111-2118). Such alteration of nucleic acid sequences encoding the penetrating peptides or modules of the invention can be carried out by standard DNA synthesis techniques.

In another embodiment, the expression vector is a yeast expression vector. Examples of vectors for expression in yeast *Saccharomyces cerivisae* include pYepSec 1 (Baldari, et al., 1987. EMBO J. 6: 229-234), pMFa (Kurjan and Herskowitz, 1982. Cell 30: 933-943), pJRY88 (Schultz et al., 1987. Gene 54: 113-123), pYES2 (Invitrogen Corporation, San Diego, Calif.), and picZ (InVitrogen Corp, San Diego, Calif.).

Alternatively, a penetrating peptide or module can be expressed in insect cells using baculovirus expression vectors. Baculovirus vectors available for expression of proteins in cultured insect cells (*e.g.,* SF9 cells) include the pAc series (Smith, et al., 1983. Mol. Cell. Biol. 3: 2156-2165) and the pVL series (Lucklow and Summers, 1989. Virology 170: 31-39).

In yet another embodiment, a nucleic acid encoding the penetrating modules of the invention is expressed in mammalian cells using a mammalian expression vector. Examples of mammalian expression vectors include pCDM8 (Seed, 1987. Nature 329: 840) and pMT2PC (Kaufman, et al., 1987. EMBO J. 6: 187-195). When used in mammalian cells, the expression vector's control functions are often provided by viral regulatory elements. For example, commonly used promoters are derived from polyoma, adenovirus 2, cytomegalovirus, and simian virus 40. For other suitable expression systems for both prokaryotic and eukaryotic cells see, *e.g.*, Chapters 16 and 17 of Sambrook, et al., MOLECULAR CLONING: A LABORATORY MANUAL. 2nd ed., Cold Spring Harbor Laboratory, Cold Spring Harbor Laboratory Press, Cold Spring Harbor, N.Y., 1989.

In another embodiment, the recombinant mammalian expression vector is capable of directing expression of the nucleic acid preferentially in a particular cell type (*e.g.,* tissue-specific regulatory elements are used to express the nucleic acid). Tissue-specific regulatory elements are known in the art. Non-limiting examples of suitable tissue-specific promoters include the albumin promoter (liver-specific; Pinkert, et al., 1987. Genes Dev. 1: 268-277), lymphoid-specific promoters (Calame and Eaton, 1988. Adv. Immunol. 43: 235-275), in particular promoters of T cell receptors (Winoto and Baltimore, 1989. EMBO J. 8: 729-733) and immunoglobulins (Banerji, et al., 1983. Cell 33: 729-740; Queen and Baltimore, 1983. Cell 33: 741-748), neuron-specific promoters (*e.g.*, the neurofilament promoter; Byrne and Ruddle, 1989. Proc. Natl. Acad. Sci. USA 86: 5473-5477), pancreas-specific promoters (Edlund, et al., 1985. Science 230: 912-916), and mammary gland-specific promoters (*e.g.,* milk whey promoter; U.S. Pat. No. 4,873,316 and European Application Publication No. 264,166). Developmentally-regulated promoters are also encompassed, *e.g.*, the murine hox promoters (Kessel and Gruss, 1990. Science 249: 374-379) and the α-fetoprotein promoter (Campes and Tilghman, 1989. Genes Dev. 3: 537-546).

The invention further provides a recombinant expression vector comprising a DNA molecule encoding the penetrating modules of the invention cloned into the expression vector in an antisense orientation. That is, the DNA molecule is operatively-linked to a regulatory sequence in a manner that allows for expression (by transcription of the DNA molecule) of an RNA molecule that is antisense to the penetrating peptide or module mRNA. Regulatory sequences operatively linked to a nucleic acid cloned in the antisense orientation can be chosen that direct the continuous expression of the antisense RNA molecule in a variety of cell types, for instance viral promoters and/or enhancers, or regulatory sequences can be chosen that direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes *see, e.g.,* Weintraub, et al., "Antisense RNA as a molecular tool for genetic analysis," Reviews-Trends in Genetics, Vol. 1(1) 1986.

Another aspect of the invention pertains to host cells into which a recombinant expression vector has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, the penetrating peptide or module can be expressed in bacterial cells such as *E. coli,* insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (*e.g.*, DNA) into a host cell, including calcium phosphate or calcium chloride co-precipitation, DEAE-dextran-mediated transfection, lipofection, or electroporation. Suitable methods for transforming or transfecting host cells can be found in Sambrook, et al. (MOLECULAR CLONING: A LABORATORY MANUAL. 2nd ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g*., resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding the penetrating peptide or module, or can be introduced on a separate vector. Cells stably transfected with the introduced nucleic acid can be identified by drug selection (*e.g*., cells that have incorporated the selectable marker gene will survive, while the other cells die).

A host cell, such as a prokaryotic or eukaryotic host cell in culture, can be used to produce (*i.e.,* express) a penetrating peptide or module. Accordingly, the invention further provides methods for producing penetrating modules using the host cells. In one embodiment, the method comprises culturing the host cell (into which a recombinant expression vector encoding a penetrating module has been introduced) in a suitable medium such that the penetrating module is produced. In another embodiment, the method further comprises isolating the penetratingmodule from the medium or the host cell.

The penetrating modules of the invention can also be produced using solid-phase peptide synthesis methods known in the art. For example, a penetrating peptide can be synthesized using the Merrifield solid-phase synthesis method. (*See e.g.,* Merrifield, R.B., J. Am. Chem. Soc. 85:2149 (1963); ENCYCLOPEDIA OF MOLECULAR BIOLOGY 806 (1st ed. 1994). In this method, the C-terminal amino acid is attached to an insoluble polymeric support resin (*e.g.*, polystyrene beads), thereby forming an immobilized amino acid. To avoid unwanted reactions as the C-terminal amino acid is attached to the resin, the amino group of the C-terminal amino acid is protected or "blocked" using, for example, a tert-butyloxylcarbonyl (t-BOC) group. The blocking group, *e.g.*, t-BOC, on the immobilized amino acid is then removed by adding a dilute acid to the solution. Before a second amino acid is attached to the immobilized peptide chain, the amino-group of the second amino acid is blocked, as described above, and the α-carboxyl group of the second amino acid is activated through a reaction with dicyclohxylcarbdiimide (DCC). The activated α-carboxyl group of the second amino acid then reacts with the free amino group of the immobilized amino acid to form a peptide bond. Additional amino acids are then individually added to the terminal amino acid of the immobilized peptide chain according to the required sequence for the desired penetrating peptide or module. Once the amino acids have been added in the required sequence, the completed peptide is released from the resin, such as for example, by using hydrogen fluoride, which does not attack the peptide bonds.

The penetrating modules of the invention can also be synthesized using Fmoc solid-phase peptide synthesis. (*See e.g.,* University of Illinois at Urbana-Champaign Protein Sciences Facility, *Solid-Phase Peptide Synthesis (SPPS), at* http://www.biotech.uiuc.edu/spps.htm). In this method, the C-terminal amino acid is attached to an insoluble polymeric support resin (*e.g.*, polystyrene beads, cross-linked polystyrene resins, etc.), such as for example, via an acid labile bond with a linker molecule. To avoid unwanted reactions as the C-terminal amino acid is being attached to the resin, the amino group of the C-terminal amino acid is blocked using an Fmoc group. The blocking group, *e.g.,* Fmoc, on the terminal amino acid of the immobilized amino acid is then removed by adding a base to the solution. Side chain functional groups are also protected using any base-stable, acid-labile groups to avoid unwanted reactions. Before the second amino acid is attached to the immobilized amino acid, the amino-group of the second amino acid is blocked, as described above, and the α-carboxyl group of each successive amino acid is activated by creating an N-hydrobenzotriazole (HOBt) ester in situ. The activated α-carboxyl group of the second amino acid and the free amino group of the immobilized amino acid then react, in the presence of a base, to form a new peptide bond. Additional amino acids are then successively added to the terminal amino acid of the immobilized peptide chain, until the desired peptide has been assembled. Once the necessary amino acids have been attached, the peptide chain can be cleaved from the resin, such as for example, by using a mixture oftrifluoroacetic acid (TFA) and scavengers (*e.g.*, phenol, thioanisol, water, ethanedithiol (EDT) and triisopropylsilan (TIS)) that are effective to neutralize any cations formed as the protecting groups attached to the side chain functional groups of the assembled peptide chain are removed.

It is well known to those skilled in the art that proteins or peptides that are produced by any of the above methods can be further chemically modified to enhance the protein half-life in the circulation. By way of non-limiting example, polyethylene glycol (PEG) residues can be attached to the penetrating peptides of the invention. Conjugating biomolecules with PEG, a process known as pegylation, is an established method for increasing the circulating half-life of proteins. Polyethylene glycols are nontoxic water-soluble polymers that, because of their large hydrodynamic volume, create a shield around the pegylated molecule, thereby protecting it from renal clearance, enzymatic degradation, as well as recognition by cells of the immune system.

Agent-specific pegylation methods have been used in recent years to produce pegylated molecules (*e.g.,* drugs, proteins, agents, enzymes, etc.) that have biological activity that is the same as, or greater than, that of the "parent" molecule. These agents have distinct in *vivo* pharmacokinetic and pharmacodynamic properties, as exemplified by the self-regulated clearance of pegfilgrastim, the prolonged absorption half-life of pegylated interferon alpha-2a. Pegylated molecules have dosing schedules that are more convenient and more acceptable to patients, which can have a beneficial effect on the quality of life of patients. (*See e.g.*, Yowell S.L. et al., Cancer Treat Rev 28 Suppl. A:3-6 (Apr. 2002)).

The invention also includes a method of contacting biological barrier with a penetrating module or peptide in an amount sufficient to enable efficient penetration between the cells. The module or peptide can be provided *in vitro, ex vivo,* or *in vivo.* Furthermore, the penetrating peptide according to this invention may be capable of potentializing the biological activity of the coupled substance. Therefore, another purpose of this invention is a method of using penetrating peptides to increase the biological activity of the effector to which it is coupled.

In addition to the peptide-effector modules or peptides and peptide-molecular vessel-effector modules, the invention also provides a pharmaceutically acceptable base or acid addition salt, hydrate, ester, solvate, prodrug, metabolite, stereoisomer, or mixture thereof. The invention also includes pharmaceutical formulations comprising a peptide-effector "module" or peptide-molecular vessel-effector module in association with a pharmaceutically acceptable carrier, diluent, protease inhibitor, surface active agent, or excipient.

Salts encompassed within the term "pharmaceutically acceptable salts" refer to non-toxic salts of the compounds of this invention which are generally prepared by reacting the free base with a suitable organic or inorganic acid to produce "pharmaceutically-acceptable acid addition salts" of the compounds described herein. These compounds retain the biological effectiveness and properties of the free bases. Representative of such salts are the water-soluble and water-insoluble salts, such as the acetate, amsonate (4,4-diaminostilbene-2, 2'-disulfonate), benzenesulfonate, benzonate, bicarbonate, bisulfate, bitartrate, borate, bromide, butyrate, calcium edetate, camsylate, carbonate, chloride, citrate, clavulariate, dihydrochloride, edetate, edisylate, estolate, esylate, fumarate, gluceptate, gluconate, glutamate, glycollylarsanilate, hexafluorophosphate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, 3-hydroxy-2-naphthoate, oleate, oxalate, palmitate, pamoate (1,1-methylene-bis-2-hydroxy-3-naphthoate, embonate), pantothenate, phosphate/diphosphate, picrate, polygalacturonate, propionate, p-toluenesulfonate, salicylate, stearate, subacetate, succinate, sulfate, sulfosaliculate, suramate, tannate, tartrate, teoclate, tosylate, triethiodide, and valerate salts.

An human patient can be treated with a pharmacologically effective amount of a module according to the present invention. The term "pharmacologically effective amount" means that amount of a drug or pharmaceutical agent (the effector) that will elicit the biological or medical response of a tissue, system, animal or human that is being sought by a researcher or clinician.

The invention also includes pharmaceutical compositions suitable for introducing an effector of interest across a biological barrier. The compositions are preferably suitable for internal use and include an effective amount of a pharmacologically active compound of the invention, alone or in combination, with one or more pharmaceutically acceptable carriers. The compounds are especially useful in that they have very low, if any, toxicity.

Preferred pharmaceutical compositions are tablets and gelatin capsules comprising the active ingredient together with a) diluents, *e.g.,* lactose, dextrose, sucrose, mannitol, sorbitol, cellulose and/or glycine; b) protease inhibitors; c) lubricants, *e.g.,* silica, talcum, stearic acid, its magnesium or calcium salt, poloxamer and/or polyethyleneglycol; for tablets also d) binders, *e.g.*, magnesium aluminum silicate, starch paste, gelatin, tragacanth, methylcellulose, sodium carboxymethylcellulose and/or polyvinylpyrrolidone; if desired e) disintegrants, *e.g.,* starches, agar, alginic acid or its sodium salt, or effervescent mixtures; and/or f) absorbents, colorants, flavors and sweeteners. Injectable compositions are preferably aqueous isotonic solutions or suspensions, and suppositories are advantageously prepared from fatty emulsions or suspensions. The compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. The compositions are prepared according to conventional mixing, granulating or coating methods, respectively, and contain about 0.01 to 75%, preferably about 0.1 to 10%, of the active ingredient.

Administration of the active compounds and salts described herein can be via any of the accepted modes of administration for therapeutic agents. These methods include systemic administration such as intravenous, or local administration such as oral, bucal, anal, bronchial, nasal, parenteral, transdermal, subcutaneous, or topical administration modes.

Depending on the intended mode of administration, the compositions may be in solid, semi-solid or liquid dosage form, such as, for example, injectables, tablets, suppositories, pills, time-release capsules, powders, liquids, suspensions, aerosol or the like, preferably in unit dosages. The compositions will include an effective amount of active compound or the pharmaceutically acceptable salt thereof, and in addition, may also include any conventional pharmaceutical excipients and other medicinal or pharmaceutical drugs or agents, carriers, adjuvants, diluents, protease inhibitors, etc., as are customarily used in the pharmaceutical sciences.

For solid compositions, excipients include pharmaceutical grades of mannitol, lactose, starch, magnesium stearate, sodium saccharin, talcum, cellulose, glucose, sucrose, magnesium carbonate, and the like may be used. The active compound defined above, may be also formulated as suppositories using for example, polyalkylene glycols, for example, propylene glycol, as the carrier.

Liquid, particularly injectable compositions can, for example, be prepared by dissolving, dispersing, etc. The active compound is dissolved in or mixed with a pharmaceutically pure solvent such as, for example, water, saline, aqueous dextrose, glycerol, ethanol, and the like, to thereby form the injectable solution or suspension.

If desired, the pharmaceutical composition to be administered may also contain minor amounts of non-toxic auxiliary substances such as wetting or emulsifying agents, pH buffering agents, and other substances such as for example, sodium acetate, triethanolamine oleate, etc.

Parental injectable administration is generally used for subcutaneous, intramuscular or intravenous injections and infusions. Injectables can be prepared in conventional forms, either as liquid solutions or suspensions or solid forms suitable for dissolving in liquid prior to injection.

One approach for parenteral administration employs the implantation of a slow-release or sustained-released system, which assures that a constant level of dosage is maintained, according to U.S. Pat. No. 3,710,795.

Those skilled in the art will recognize that the penetrating modules of the instant invention can be used as an oral vaccine. Such a vaccine may comprise a penetrating peptide coupled with a desired antigenic sequence, including, but not limited to, the PA antigen of Anthrax. This fusion protein is then orally administered to a subject in need of vaccination.

An "antigen" is a molecule or a portion of a molecule capable of stimulating an immune response, which is additionally capable of inducing an animal or human to produce antibody capable of binding to an epitope of that antigen. An "epitope" is that portion of any molecule capable of being recognized by and bound by a major histocompatability complex ("MHC") molecule and recognized by a T cell or bound by an antibody. A typical antigen can have one or more than one epitope. The specific recognition indicates that the antigen will react, in a highly selective manner, with its corresponding MHC and T cell, or antibody and not with the multitude of other antibodies which can be evoked by other antigens.

A peptide is "immunologically reactive" with a T cell or antibody when it binds to an MHC and is recognized by a T cell or binds to an antibody due to recognition (or the precise fit) of a specific epitope contained within the peptide. Immunological reactivity can be determined by measuring T cell response *in vitro* or by antibody binding, more particularly by the kinetics of antibody binding, or by competition in binding using known peptides containing an epitope against which the antibody or T cell response is directed as competitors.

Techniques used to determine whether a peptide is immunologically reactive with a T cell or with an antibody are known in the art. Peptides can be screened for efficacy by *in vitro* and *in vivo* assays. Such assays employ immunization of an animal, *e.g.*, a mouse, a rabbit or a primate, with the peptide, and evaluation of the resulting antibody titers.

Also included within the invention are vaccines that can elicit the production of secretory antibodies (IgA) against the corresponding antigen, as such antibodies serve as the first line of defense against a variety of pathogens. Oral vaccination, which has the advantages of being a non-invasive route of administration, is the preferred means of immunization for obtaining secretory antibodies.

The compounds of the present invention can be administered in such oral dosage forms as tablets, capsules (each including timed release and sustained release formulations), pills, powders, granules, elixirs, tinctures, suspensions, syrups and emulsions. Likewise, they may also be administered in intravenous (both bolus and infusion), intraperitoneal, subcutaneous or intramuscular form, and all using forms well known to those of ordinary skill in the pharmaceutical arts.

The dosage regimen utilizing the compounds is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to prevent, counter or arrest the progress of the condition.

Oral dosages of the present invention, when used for the indicated effects, may be provided in the form of scored tablets containing 0.005, 0.01, 0.025, 0.05, 0.1, 0.25, 0.5, 1.0, 2.5, 5.0, 10.0, 15.0, 25.0, 50.0, 100.0, 250.0, 500.0 or 1000.0 mg of active ingredient.

Compounds of the present invention may be administered in a single daily dose, or the total daily dosage may be administered in divided doses of two, three or four times daily. Furthermore, preferred compounds for the present invention can be administered in bucal form via topical use of suitable bucal vehicles, bronchial form via suitable aerosols or inhalants, intranasal form via topical use of suitable intranasal vehicles, or via transdermal routes, using those forms of transdermal skin patches well known to those of ordinary skill in that art. To be administered in the form of a transdermal delivery system, the dosage administration will, of course, be continuous rather than intermittent throughout the dosage regimen. Other preferred topical preparations include creams, ointments, lotions, aerosol sprays and gels, wherein the concentration of active ingredient would range from 0.1% to 15%, w/w or w/v.

The compounds herein described in detail can form the active ingredient, and are typically administered in admixture with suitable pharmaceutical diluents, excipients or carriers (collectively referred to herein as "carrier" materials) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders, lubricants, protease inhibitors, disintegrating agents and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, poloxamer, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methylcellulose, agar, bentonite, xanthan gum and the like.

The compounds of the present invention can also be administered in the form of liposome delivery systems, such as small unilamellar vesicles, large unilamellar vesicles and multilamellar vesicles. Liposomes can be formed from a variety of phospholipids, containing cholesterol, stearylamine or phosphatidylcholines. In some embodiments, a film of lipid components is hydrated with an aqueous solution of drug to a form lipid layer encapsulating the drug, as described in U.S. Pat. No. 5,262,564.

The compounds of the present invention may also be coupled with soluble polymers as targetable drug carriers. Such polymers can include polyvinylpyrrolidone, pyran copolymer, polyhydroxypropyl-methacrylamide-phenol, polyhydroxyethylaspanamidephenol, or polyethyleneoxidepolylysine substituted with palmitoyl residues. Furthermore, the compounds of the present invention may be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates and cross-linked or amphipathic block copolymers of hydrogels.

Any of the above pharmaceutical compositions may contain 0.01-99%, preferably 0.1-10% of the active compounds as active ingredients.

Following is a description by way of example only with reference to the accompanying drawings of embodiments of the present invention.

In the drawings:

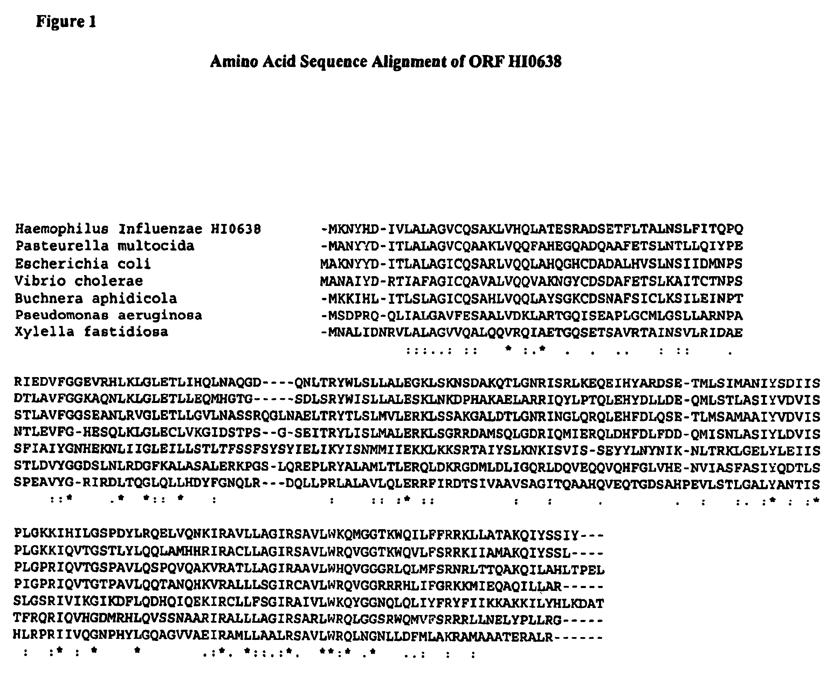

Figure 1 shows amino acid sequence alignment of ORF HI0638 and its homologs from other pathogenic bacteria.

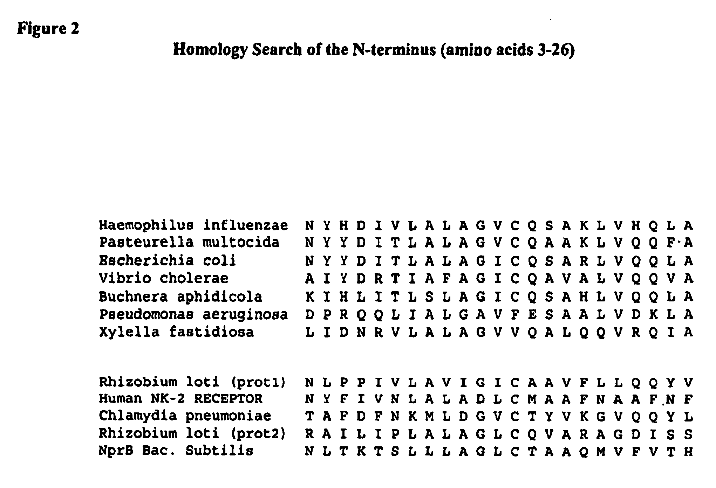

Figure 2 shows amino acid sequence alignment of the peptides used in this invention as penetrating modules as well as their organism of origin.

### EXAMPLES

### Example 1. Demonstration of the efficacy of the penetrating module to enable the translocation of a peptide through an epithelial barrier.

The tested peptide, representing a penetrating peptide and a radio-labeled probe in a contiguous construct of SEQ ID NO 3 and SEQ ID NO 17, hereby named IBW 002 (Novetide, lot 40139-45, sequence: Ac-N-Y-Y-D-I-T-L-A-L-A-G-I-C-Q-S-A-R-L-V-Q-Q-L-A-G-G-G-K-G-G-K-NH₂ (SEQ ID NO:22), was ¹²⁵I-labeled using the Bolton Hunter Reagent (Biochem. J. 133:529-39 (1973)). The labeled peptide was loaded on a Sephadex G10 column, washed with phosphate buffer and eluted with 3M Tiocyanate in DDW + 50% n-butanol. The peak, containing 6 fractions, was kept and used for in *vivo* experiments.

The test sample was prepared as shown in Table 2

**Table 2**

| | |
|---|---|
| ¹²⁵I-IBW-002 sample | 26ul |
| PE 6,200 | 20ul |
| Aprotinin | 100ul |
| PB | 849ul |
| NAC (stock 9.8mg/ml) | 5ul |

### In vivo experimental procedure:

Four male BALB/c mice, 9-10 weeks old, were deprived of food, 18 hours prior to the experiment.

The mice were anesthetized by i.p. injection of 0.05ml of a mixture of 0.15ml xylazine + 0.85ml of ketamin. A 2cm long incision was made along the center of the abdomen, through the skin and abdominal wall. An intestine loop was gently pulled out through the incision and placed on wet gauze beside the animal. The loop remained intact through the entire procedure and was kept wet during the whole time. The tested compound was injected into the loop, 0.2ml/ mouse, containing around 200,000 cpm, using a 26G needle. Fifteen minutes post injection the tip of the tail was cut and a 50µl blood sample was drawn into a glass capillary. This was repeated 30 and 60 minutes post injection. The animals were subsequently sacrificed. The following organs were removed: epididymal fat, kidneys, liver, lungs and brain. Radioactivity in each organ and in blood samples was counted. A 5µl sample of the injected solution was counted to determine the total injected cpm per mouse.

### Results:

The total radioactive labeling injected per mouse was 224,160 cpm. The cpm recovered from each tissue is summarized in Table 3. Total cpm in the blood was calculated based on the 50µl blood sample drawn out at different time points post injection, in the assumption that total blood volume of each mouse is 2ml.

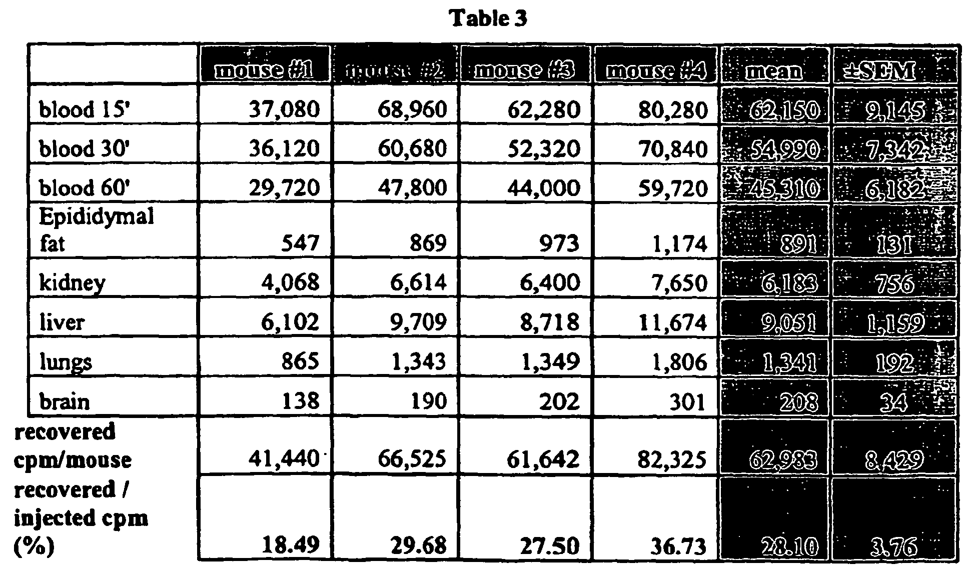

The percent of ¹²⁵I-IBW-002 in the blood relative to the total injected ¹²⁵I-IBW-002, at the 3 different time points, is presented in Table 4.

As can be seen in Table 4, the relative amount of ¹²⁵I-IBW-002 in the blood peaked already at 15 min. post-intestinal administration and decreased with time. This probably indicates peptide distribution from the blood to various organs.

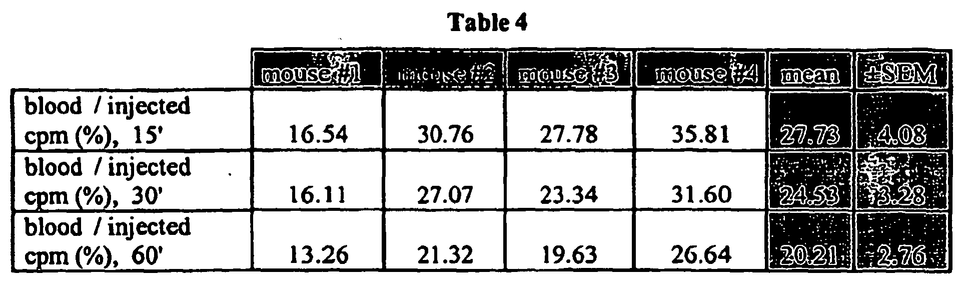

In similar experiments, a control ¹²⁵I-labeled peptide (SEQ ID NO:17), lacking the penetrating peptide-sequence, was injected into a mouse intestinal loop. The average amount of the control peptide recovered from the blood and various organs was only about 1/5 of that obtained with the full conjugate of the IBW-002 peptide, although this control peptide is about ¼ in its size as compared with IBW-002.

### Example 2. Development of optimal formulation.

To reach an improved formulation, a number of compounds were tested in experiments similar to that described in Example 1, with the following change: urine was collected and the measured radioactivity was added to that of the recovered tissues.

### a. Non ionic detergents (preferably Poloxamers)

**Table 5. Initial formulation with Pluronic PE 6,200**

| | |
|---|---|
| **Peptide sample (radiolabeled)** | 10ul |
| **PE 6,200** | 20ul |
| **Aprotinin** | 100ul |
| **PB** | 5ul |
| **NAC (9.8mg/ml)** | 5ul |

The total radioactive labeling injected per mouse was 125,320 cpm. The cpm recovered from each tissue is summarized in Table 6. Total cpm in the blood was calculated based on the 50µl blood sample drawn out at different time points post injection, in the assumption that total blood volume of each mouse is 2ml.

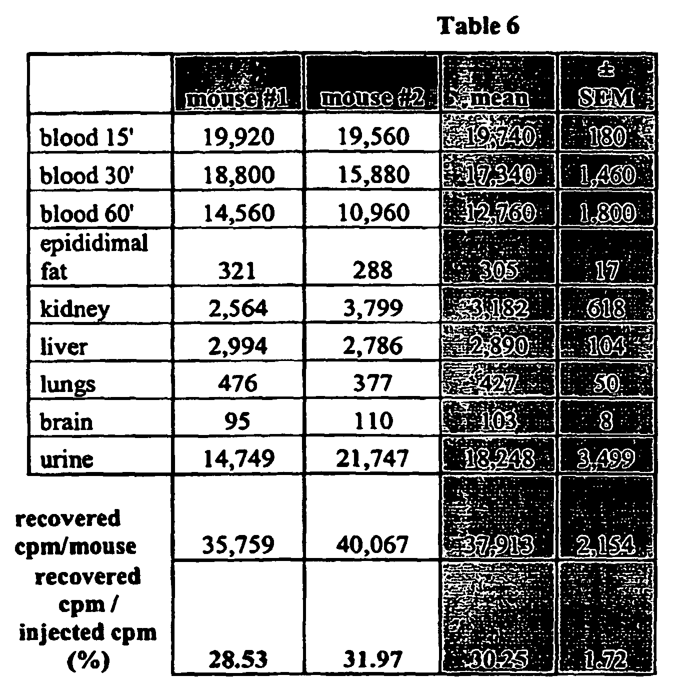

The percent of ¹²⁵I-IBW-002 in the blood relative to the total injected ¹²⁵I-IBW-002, at the 3 different time points, is presented in Table 7

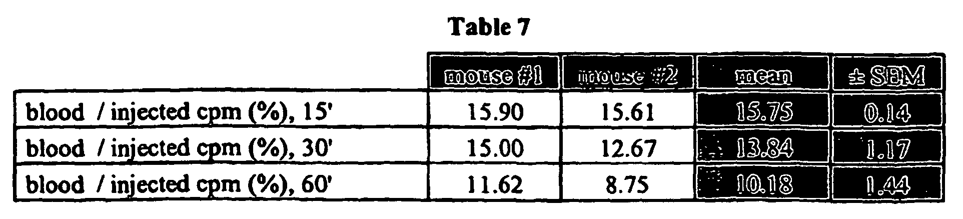

Calculated total recovery was **30.25%**.

### b. Bile salts (preferably Taurodeoxycholic acid)

**Table 8.**

| | |
|---|---|
| **Peptide sample (radiolabeled)** | 10ul |
| **PE 6,200** | 20ul |
| **Aprotinin** | 100ul |
| **PB** | 802.5ul |
| **NAC (9.8mg/ml)** | 5ul |
| **Taurodeoxycholic acid (8% in DDW)** | 62.5ul |

The total radioactive labeling injected per mouse was 195,900 cpm. The cpm recovered from each tissue is summarized in Table 9. Total cpm in the blood was calculated based on the 50µl blood sample drawn out at different time points post injection, in the assumption that total blood volume of each mouse is 2ml.

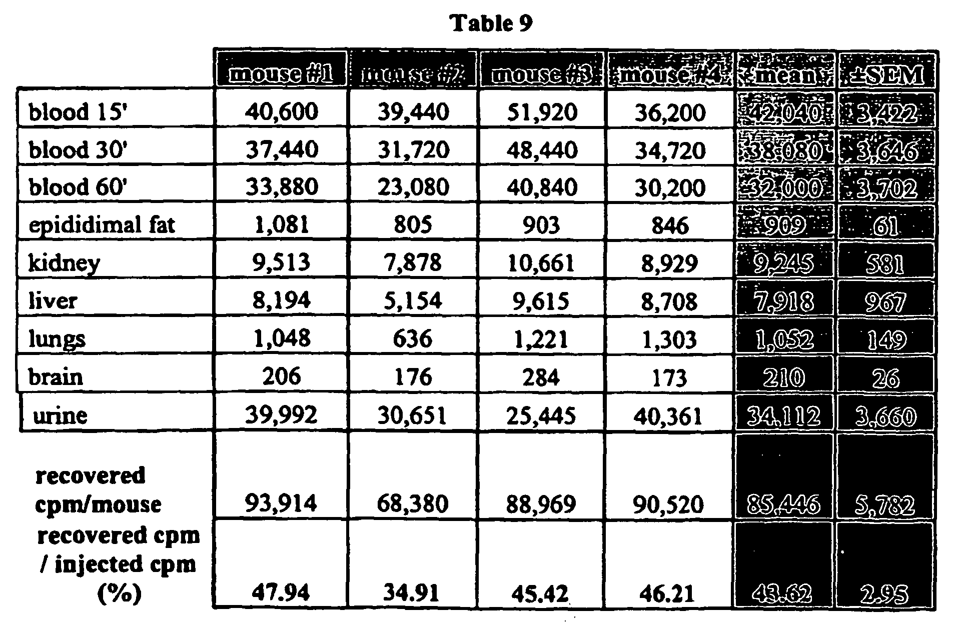

The percent of ¹²⁵I-IBW-002 in the blood relative to the total injected ¹²⁵I-IBW-002, at the 3 different time points, is presented in Table 10.

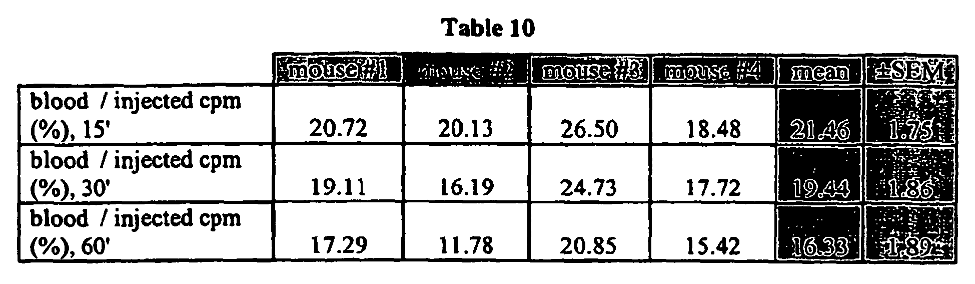

Calculated total recovery was **43.62%.**

### c. Pluronic F-68 as a preferred Poloxamer

**Table 11.**

| | |
|---|---|
| **Peptide sample (radiolabeled)** | 18ul |
| **Pluronic F.68 (2.72% in PB)** | 735ul |
| **Aprotinin** | 100ul |
| **PB** | 80ul |
| **NAC (9.8mg/ml)** | 5ul |
| **Taurodeoxycholic acid (8% in DDW)** | 62.5ul |

The total radioactive labeling injected per mouse was 143,000 cpm. The cpm recovered from each tissue is summarized in Table 12. Total cpm in the blood was calculated based on the 50µl blood sample drawn out at different time points post injection, in the assumption that total blood volume of each mouse is 2ml.

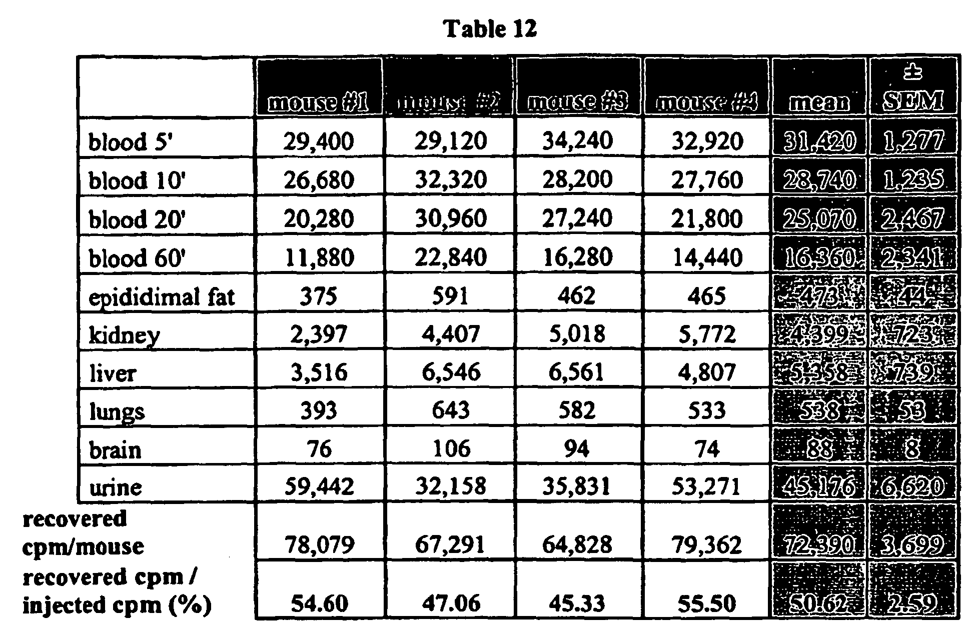

The percent of ¹²⁵I-IBW-002 in the blood relative to the total injected ¹²⁵I-IBW-002, at the four different time points, is presented in Table 13.

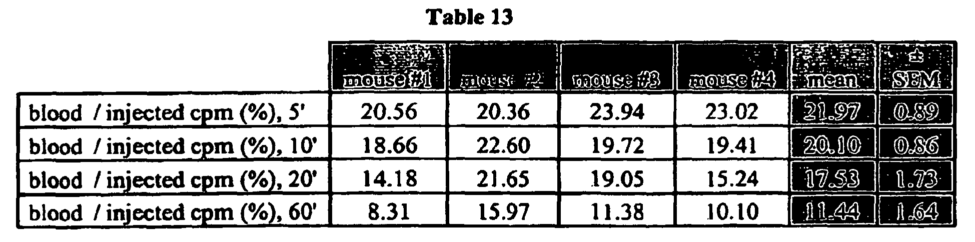

Calculated total recovery was **50.62%.**

**Table 14: Summary of formulation results regarding the added detergents**

| **Formulation** | **Recovery (%)** |
|---|---|
| 2% PE 6,200 | 30.25 |
| 2% PE 6,200 + 0.5% Taurodeoxycholic acid | 43.62 |
| 2% Pluronic F-68 + 0.5% Taurodeoxycholic acid | 50.62 |

### Example 3. Relative potency of penetrating peptides as tested in mice.

**Table 15**

| **Peptide's name** | **SEQ ID NO.** | **Sequence** | **Relative potency (%)** |
|---|---|---|---|
| IBW-002 | 22 | AcNYYDITLALAGICQSARLVQQLAGGGKGGKNH₂ | 100 |
| IBW-002V2 | 36 | AcMYYDITLALAGICQSARLVQQLAGGGKGGKNH₂ | 80 |
| IBW-002V3 | 37 | AcMYDITLALAGICQSARLVQQLAGGGKGGKNH₂ | 77 |
| IBW-006 | 33 | AcNYHDWLALAGVCQSARLVHQLAGGKGGKNH₂ | 77 |
| IBW-002V1 | 35 | AcMNYYDITLALAGICQSARLVQQLAGGGKGGKNH₂ | 73 |
| IBW-007 | 34 | AcNYFLVNLAFAEASMAAFNTVVNFGGKGGKNH₂ | 66 |
| IBW-004 | 31 | AcNYFIVNLALADLCMAAFNAAFNFGGGKGGKNH₂ | 63 |
| IBW-005 | 32 | AcNERNLTRTSLLLAGLCTAAQMVFVGGGKGGKNH₂ | 51 |
| IBW-003 | 30 | AcNLPPIVLAVIGICAAVFLLQQYVGGGKGGKNH₂ | 37 |

### Example 4. Conditionally-activated effectors: Inhibition of excessive blood coagulation.

SEQ ID NO:3 (or any other sequence from SEQ ID NO:1-15, 24-29) is coupled to a coagulation inhibitory protein (*e.g.*, hirudin, hirulog, Protein C, Protein C activator or Protein S) or a thrombolysis activating protein (*i.e.*, tPA) through the sequence IEGR (SEQ ID NO:16), a cleavage site recognized by factor Xa.

At sites of excessive blood coagulation, the linker sequence will be cleaved by the abundant factor Xa, thereby releasing the coupled coagulation inhibitory protein. As a result, the coagulation process will be attenuated. Inhibition of excessive or pathological coagulation can be assessed locally by reduction in thrombus size, morphology, and propagation along the vasculature.

Consequently, a reduction of ischemic damage can be demonstrated via histological and functional extent of an infarct. Systemic effects can be assessed by lengthened Prothromin Time (PT) and Partial Thromboplastin Time (PTT), and reduced levels of fibrinogen degradation products and D- dimers (FDPs).

### Example 5. Conditionally-activated effectors: Inhibition of pathologic inflammatory processes.

SEQ ID NO:3 (or any other sequence from SEQ ID NO:1-15, 24-29) is coupled to a complement inhibitory protein (*e.g.*, a Clq inhibitor) through a cleavage site that is recognized by complement cascade proteins (*e.g.,* C3a).

At instances of pathological complement activation, such as excessive inflammatory processes, the complement inhibitor will be released. Inhibition of the complement cascade can be assessed directly by a reduction in CH50, or individual C3 and C4 activity. Overall modulation of inflammation may be determined by a reduction in levels of acute phase proteins, *e.g.*, C-reactive protein (CRP), or a reduction in elevated sedimentation rate (ESR).

### Example 6: Utilization of the penetrating module for oral vaccination.

SEQ ID NO: 3 (or any other sequence from SEQ ID NO:1-15, 24-29) is coupled to a desired antigenic sequence. For example, a fusion protein composed of the penetrating module coupled to the PA antigen of Anthrax. Such a fusion protein can be administered to a subject in need of vaccination.

This method allows simple and rapid vaccination of large populations in need thereof. Another advantage of this method is the production of high titers of IgA antibodies and the subsequent presence of IgA antibodies in the epithelial mucosa, which are the sites of exposure to antigens.

Efficacy of vaccination can be demonstrated by the measurement of specific antibody titers, especially for IgA, as well as the measurement of immunological response to stimulation, such as for example, via a cutaneous hyerpsensitivity reaction in response to subcutaneous administration of antigen.

### Example 7: Utilization of the penetrating module for bulk translocation.

By covalently attaching stearyl residues to the free amino groups of the two lysines at the C-terminus of SEQ ID NO:22, a hydrophobized penetrating peptide is produced. This hydrophobized penetrating peptide is then incorporated into the interface of taxol-containing microparticles composed of triglyceride medium.

This method allows for the translocation of highly hydrophobic drugs, such as taxol, from the intestine into the bloodstream. Because such drugs cannot be otherwise absorbed from the intestine, they are typically administered to patients via invasive means.

The effects oftaxol-containing microparticles can be demonstrated by a reduction in tumor size, metastases or other tumor-related markers.

### Example 8. Recombinant human insulin (rh-Insulin) delivery across mucosal epithelia via a fusion construct

SEQ ID NO:3 (or any other sequence from SEQ ID NO:1*-*15, 24-29) is coupled to rh-Insulin in one of two ways:
1. through a cleavage site:
   NYYDITLALAGICQSARLVQQLA-GG-IEGR- rh-Insulin (SEQ ID NO: 18)
2. without a cleavage site:
   NYYDITLALAGICQSARLVQQLA-GG- rh-Insulin (SEQ ID NO:19).

The resulting compound is dissolved in a 2% pluronic PE 6200 aqueous solution. Two hundred µL of this solution, (or vehicle alone), is injected into an intestinal loop of a mouse and blood glucose levels are subsequently measured.

Blood glucose levels decrease in relation to the amount of insulin absorbed from the intestine into the bloodstream (*i.e.,* in an amount that correlates to the amount of insulin absorbed). Thus, this drug delivery system can replace the need for insulin injections, thereby providing an efficient, safe and convenient route of administration for diabetes patients.

### Example 9. Recombinant human insulin (rh-Insulin) delivery across mucosal epithelia via a molecular vessel fusion construct

SEQ ID NO:3 (or any other sequence from SEQ ID NO:1-15, 24-29) is coupled to a ligand binding domain of the insulin receptor (or minireceptor) which, in turn, is bound to rh-Insulin as follows:
NYYDITLALAGICQSARLVQQLA-GG- linearized insulin receptor (SEQ ID NO:23) (see Kristensen, et al., J. Biol. Chem., 274(52):37351-56 (1999)) plus an equimolar amount of rh-insulin.

The resulting complex is dissolved in a 2% pluronic PE 6200 aqueous solution. Two hundred µL of the above dissolved complex, (or vehicle alone), is injected into an intestinal loop of a mouse and blood glucose levels are subsequently measured.

Blood glucose levels decrease in relation to the amount of insulin absorbed from the intestine into the bloodstream (*i.e.*, in an amount that correlates to the amount of insulin absorbed). Thus, this drug delivery system can replace the need for insulin injections, thereby providing an efficient, safe and convenient route of administration for diabetes patients.

### Example 10. Low molecular weight heparin delivery across mucosal epithelia

SEQ ID NO:3 (or any other sequence from SEQ ID NO:1-15, 24-29) is coupled to heparin for example, through the free amino group of an extra lysine added at the C-terminus, in one of two ways:
1. through a cleavage site:
   NYYDITLALAGICQSARLVQQLA-GG-IEGR-K- heparin (SEQ ID NO:20)
2. without a cleavage site:
   NYYDITLALAGICQSARLVQQLA-GG-K- heparin (SEQ ID NO:21).

The resulting compound is dissolved in a 2% pluronic PE 6200 aqueous solution. Two hundred µL of this solution, (or vehicle alone), is injected into an intestinal loop of a mouse. Partial Thrombin Time (PTT) values are subsequently measured.

Partial Thrombin Time (PTT) values decrease in relation to the amount of heparin absorbed from the intestine loop into the bloodstream (*i.e.*, in an amount that correlates to the amount of insulin absorbed). Therefore, this drug delivery system will replace the use of heparin injections.

### OTHER EMBODIMENTS

From the foregoing detailed description of the specific embodiments of the invention, it should be apparent that unique methods of translocation across epithelial and endothelial barriers have been described. Although particular embodiments have been disclosed herein in detail, this has been done by way of example for purposes of illustration only.

## Claims

1. A penetrating module comprising a penetrating peptide and an effector that is coupled or fused to said penetrating peptide, said penetrating peptide consisting of at least one amino acid sequence selected from:
(a) SEQ ID NOS: 1-15; and
(b) SEQ ID NOS: 24-29;
which penetrating peptide is capable of translocating across a biological barrier.

2. A penetrating module as claimed in claim 1, wherein the amino acid sequence is SEQ ID NO: 3, SEQ ID NO: 8, SEQ ID NO: 9, SEQ ID NO: 12 or SEQ ID NO: 24.

3. A penetrating module as claimed in claim 1, wherein said effector is a bioactive peptide, selected from the group consisting of insulin, growth hormone, a gonadotropin, a growth factor, erythropoietin, granulocyte/monocyte colony stimulating factor (GM-CSF), αMSH, enkephalin, dalargin, kyotorphin, EPO, bFGF, hirulog, a lutenizing hormone releasing hormone (LHRH) analog, and neurotrophic factors.

4. A penetrating module as claimed in claim 1, wherein said effector is a pharmaceutically active agent, selected from the group consisting of an anticoagulant, a toxin, an antibiotic, an antipathogenic agent, an antigen, an antibody fragment, an immunomodulator, a vitamin, an enzyme, an antineoplastic agent, and a therapeutic agent.

5. An *ex vivo* method of translocating a penetrating peptide across a biological barrier, wherein the penetrating peptide is a penetrating peptide as claimed in claim 1 or claim 2.

6. A penetrating module as claimed in claim 1, wherein translocation across a biological barrier occurs within a tissue selected from epithelial cells and endothelial cells.

7. A penetrating module as claimed in claim 1, wherein said biological barrier is selected from tight junctions and the plasma membrane, and wherein said tight junctions are selected from mucosal epithelia, intestinal epithelia, respiratory epithelia, and vascular epithelia.

8. A penetrating module as claimed in claim 7, wherein the vascular epithelia includes the blood-brain barrier.

9. A pharmaceutical composition comprising a therapeutically or prophylactically effective amount of a penetrating module as claimed in claim 1, and a pharmaceutically acceptable carrier.

10. A pharmaceutical composition as claimed in claim 9, wherein the composition further comprises a mixture of at least two substances selected from a non-ionic detergent, an ionic detergent, a protease inhibitor, and a reducing agent.

11. A pharmaceutical composition as claimed in claim 10, wherein the non-ionic detergent is a poloxamer, optionally pluronic^{®} F-68.

12. A pharmaceutical composition as claimed in claim 10, wherein the ionic detergent is a bile salt, optionally taurodeoxycholate.

13. A pharmaceutical composition as claimed in claim 10, wherein the protease inhibitor is selected from aprotinin and soy bean trypsin inhibitor.

14. A pharmaceutical composition of claim 10, wherein the reducing agent is N-Acetyl-L-Cysteine (NAC).

15. A method of producing a penetrating module as claimed in claim 1 comprising coupling said effector to said penetrating peptide.

16. A method as claimed in claim 15, wherein the coupling of said effector is achieved by a covalent bond.

17. A method as claimed in claim 16, wherein said covalent bond is a peptide bond.

18. A method as claimed in claim 16, wherein the covalent bond is achieved by a homo- or a hetero-functional bridging reagent, and wherein the bridging reagent is optionally a succinimidyl-(N-maleimidomethyl) cyclohexane-1-carboxylate (SMCC)-type reagent.

19. A method as claimed in claim 16, wherein the covalent bond is achieved by a peptide linker, which optionally has the sequence of SEQ ID NO: 16 or SEQ ID NO:17, or which can optionally be cleaved by an enzyme.

20. A method as claimed in claim 19, wherein the peptide linker is designed to be cleaved by an enzyme conditionally activated under a certain physiological state and wherein the released effector favorably influences said physiological state.

21. A method as claimed in claim 15, wherein the coupling of said effector is achieved by a non-covalent bond.

22. A method as claimed in claim 21, wherein the non-covalent bond is achieved by an attachment of a hydrophobic moiety to the penetrating peptide, wherein the hydrophobic moiety enables the penetrating module to be incorporated at the interface of a hydrophobic vesicle in which the effector is contained.

23. A method as claimed in claim 21, wherein the non-covalent bond is the result of a biotin-avidin or biotin-streptavidin interaction.

24. An *ex vivo* method of translocating an effector across a biological barrier, said method comprising:
a) coupling said elector to a penetrating peptide to form a penetrating module as claimed in claim 1; and
b) introducing said penetrating module to the biological barrier.

25. Use of a penetrating module as claimed in claim 1 in the manufacture of a vaccine for oral vaccination for administration to a subject, the penetrating peptide being coupled with a desirable antigen.

26. Use of a pharmaceutical composition as claimed in claim 9 in the manufacture of a medicament for administration to a subject for treating or preventing a disease or pathological condition, the pharmaceutical composition being used in an amount sufficient to treat or prevent said disease or said pathological condition in said subject.

27. A use as claimed in claim 26, wherein said disease or said pathological condition is selected from endocrine disorders, diabetes, infertility, hormone deficiencies, osteoporosis, neurodegenerative disorders, Alzheimer's disease, Parkinson's disease, Huntington's disease, cardiovascular disorders, atherosclerosis, hypercoagulable states, hypocoagulable states, coronary disease, cerebrovascular events, metabolic disorders, obesity, vitamin deficiencies, haematological disorders, and neoplastic disease.

28. A method for producing a penetrating module as claimed in claim 1, said method comprising
a) transfecting a production cell with a vector comprising a nucleic acid molecule of a fusion protein encoding said penetrating peptide and an effector operably linked to an expression control sequence;
b) culturing said production cell under conditions that permit production of a fusion protein consisting of the penetrating peptide and an effector peptide; and
c) isolating said fusion protein.

29. A penetrating module as claimed in claim 1, wherein said penetrating peptide is fused to a molecular vessel, which molecular vessel encloses an effector.

30. A penetrating module as claimed in claim 29, wherein the effector is selected from a bioactive peptide and a pharmaceutically active agent.

31. A penetrating module as claimed in claim 30, wherein said bioactive peptide is selected from insulin, growth hormone, a gonadotropin, a growth factor, erythropoietin, GM-CSF, αMSH, enkephalin, dalargin, kyotorphin, EPO, bFGF, hirulog, an LHRH analog, and neurotrophic factors.

32. A penetrating module as claimed in claim 31, wherein said pharmaceutically active agent is selected from an anticoagulant, a toxin, an antibiotic, an antipathogenic agent, an antigen, an antibody fragment, a vitamin, an immunomodulator, an enzyme, an antineoplastic agent, heparin, methotraxate, and a therapeutic agent.

33. A penetrating module as claimed in claim 29, wherein the molecular vessel is selected from a soluble receptor, a minireceptor, and a binding protein.

34. A penetrating module as claimed in claim 33, wherein the soluble receptor is a soluble insulin receptor, or wherein the minireceptor is the ligand-binding domain of the insulin receptor, or wherein the binding protein is Intrinsic factor.

35. A penetrating module as claimed in claim 33, wherein the effector is vitamin B12, or a penetrating module as claimed in claim 34, wherein the effector is insulin.

36. A pharmaceutical composition comprising a therapeutically or prophylactically effective amount of a penetrating module as claimed in claim 29, and a pharmaceutically acceptable carrier.

37. A method for producing a penetrating module as claimed in claim 1, said method comprising using solid-phase synthesis to construct the peptide and fusing said effector to the peptide.

38. A penetrating module as claimed in claim 1, further comprising one or more polyethylene glycol residues attached to the penetrating peptide.

39. A method as claimed in claim 28, wherein the fusion protein further comprises one or more polyethylene glycol residues attached to the fusion protein.

## Patentansprüche

1. Durchdringungsmodul umfassend eine Durchdringungspeptid und einen Effektor, der mit dem Durchdringungspeptid gekoppelt oder fusioniert ist, das Durchdringungspeptid besteht aus mindestens einer Aminosäuresequenz, die ausgewählt ist aus
(a) SEQ ID NRN: 1-5; und
(b) SEQ ID NRN: 24-29;
wobei das Durchdringungspeptid in der Lage ist, über eine biologische Barriere zu translozieren.

2. Durchdringungsmodul wie in Anspruch 1 beansprucht, wobei die Aminosäuresequenz SEQ ID NR: 3, SEQ ID NR: 8, SEQ ID NR: 9, SEQ ID NR: 12 oder SEQ ID NR: 24 ist.

3. Durchdringungsmodul wie in Anspruch 1 beansprucht, wobei der Effektor ein bioaktives Peptid ist, das aus der Gruppe ausgewählt ist, die aus Insulin, Wachstumshormon, einem Gonadotropin, einem Wachstumsfaktor, Erythropoietin, Granulozyten/Monozyten Koloniestimulierendem Faktor (GM-CSF), α-MSH, Enkephalin, Dalargin, Kyotorphin, EPO, bFGF, Hirulog, einem luteinisierenden Hormon freisetzenden Hormon (LHRL) Analog und neutrotrophen Faktoren besteht.

4. Durchdringungsmodul wie in Anspruch 1 beansprucht, wobei der Effektor ein pharmazeutisch aktives Mittel ist, das ausgewählt ist aus der Gruppe, die aus einem Antikoagulans, einem Toxin, einem Antibiotikum, einem antipathogenen Mittel, einem Antigen, einem Antikörperfragment, einem Immunmodulator, einem Vitamin, einem Enzym, einem antineoplastischen Mittel und einem therapeutischen Mittel besteht.

5. *Ex vivo* Verfahren zum Translozieren eines Durchdringungspeptids über eine biologische Barriere, wobei das Durchdringungspeptid ein Durchdringungspeptid wie in Anspruch 1 oder Anspruch 2 beansprucht, ist.

6. Durchdringungsmodul wie in Anspruch 1 beansprucht, wobei die Translokation über eine biologische Barriere innerhalb eines Gewebes stattfindet, das ausgewählt ist aus e-pithelialen Zellen und endothelialen Zellen.

7. Durchdringungsmodul wie in Anspruch 1 beansprucht, wobei die biologische Barriere ausgewählt ist aus Tight Junctions und der Plasmamembran, und wobei die Tight Junctions ausgewählt sind aus Schleimhautepithel, Darmepithel, Atemepithel und Gefäßepithel.

8. Durchdringungsmodul wie in Anspruch 7 beansprucht, wobei das Gefäßepithel die Bluthimschranke einschließt.

9. Pharmazeutische Zusammensetzung umfassend eine therapeutisch oder prophylaktisch wirksame Menge eines Durchdringungsmoduls wie in Anspruch 1 beansprucht, und einen pharmazeutisch verträglichen Träger.

10. Pharmazeutische Zusammensetzung wie in Anspruch 9 beansprucht, wobei die Zusammensetzung weiterhin eine Mischung aus mindestens zwei Substanzen umfasst, die ausgewählt sind aus einem nichtionischen Detergens, einem ionischen Detergens, einem Proteaseinhibitor und einem Reduktionsmittel.

11. Pharmazeutische Zusammensetzung wie in Anspruch 10 beansprucht, wobei das nichtionische Detergens ein Poloxamer, wahlweise Pluronic^{®} F-68 ist.

12. Pharmazeutische Zusammensetzung wie in Anspruch 10 beansprucht, wobei das ionische Detergens ein Gallensalz, wahlweise Taurodesoxycholat ist.

13. Pharmazeutische Zusammensetzung wie in Anspruch 10 beansprucht, wobei der Proteaseinhibitor ausgewählt ist aus Aprotinin und Sojabohnen Trypsininhibitor.

14. Pharmazeutische Zusammensetzung nach Anspruch 10, wobei das Reduktionsmittel N-Acetyl-L-cystein (NAC) ist.

15. Verfahren zur Herstellung eines Durchdringungsmoduls wie in Anspruch 1 beansprucht, umfassend das Koppeln des Effektors an das Durchdringungspeptid.

16. Verfahren wie in Anspruch 15 beansprucht, wobei das Koppeln des Effektors durch eine kovalente Bindung erreicht wird.

17. Verfahren wie in Anspruch 16 beansprucht, wobei die kovalente Bindung eine Peptidbindung ist.

18. Verfahren wie in Anspruch 16 beansprucht, wobei die kovalente Bindung durch ein homo- oder ein heterofunktionelles Brückenreagens erreicht wird, und wobei das Brückenreagens wahlweise ein Reagens des Typs eines Succinimidyl-(N-maleimidomethyl)-cyclohexan-1-carboxylats (SMCC) ist.

19. Verfahren wie in Anspruch 16 beansprucht, wobei die kovalente Bindung durch einen Peptidlinker erreicht wird, der wahlweise die Sequenz von SEQ ID NR: 16 oder SEQ ID NR: 17 aufweist, oder der wahlweise durch ein Enzym gespalten werden kann.

20. Verfahren wie in Anspruch 19 beansprucht, wobei der Peptidlinker derart ausgestaltet ist, das er durch ein Enzym gespalten wird, dass konditionell unter einem bestimmten physiologischen Zustand aktiviert wird, und wobei der freigesetzte Effektor den physiologischen Zustand vorteilhaft beeinflusst.

21. Verfahren wie in Anspruch 15 beansprucht, wobei das Koppeln des Effektors durch eine nicht-kovalente Bindung erreicht wird.

22. Verfahren wie in Anspruch 21 beansprucht, wobei die nicht-kovalente Bindung durch eine Anheftung eines hydrophoben Rests an das Durchdringungspeptid erreicht wird, wobei der hydrophobe Rest es dem Durchdringungsmodul erlaubt, in den Zwischenraum eines hydrophoben Vesikels, in dem der Effektor enthalten ist, eingebaut zu werden.

23. Verfahren wie in Anspruch 21 beansprucht, wobei die nicht-kovalente Bindung das Ergebnis einer Biotin-Avidin oder Biotin-Streptavidin Interaktion ist.

24. *Ex vivo* Verfahren zum Translozieren eines Effektors über eine biologische Barriere, wobei das Verfahren umfasst:
a) Koppeln des Effektors an ein Durchdringungspeptid um ein Durchdringungsmodul, wie in Anspruch 1 beansprucht, zu bilden; und
b) Einführen des Durchdringungsmoduls in die biologische Barriere.

25. Verwendung eines Durchdringungsmoduls wie in Anspruch 1 beansprucht in der Herstellung eines Impfstoffs für orale Impfung für die Verabreichung an ein Subjekt, wobei das Durchdringungspeptid mit einem gewünschten Antigen gekoppelt ist.

26. Verwendung einer pharmazeutischen Zusammensetzung wie in Anspruch 9 beansprucht in der Herstellung eines Medikaments zur Verabreichung an ein Subjekt zum Behandeln oder Verhindern einer Erkrankung oder eines pathologischen Zustandes, wobei die pharmazeutische Zusammensetzung in einer Menge verwendet wird, die ausreichend ist, um die Erkrankung oder den pathologischen Zustand in dem Subjekt zu behandeln oder zu verhindern.

27. Verwendung wie in Anspruch 26 beansprucht, wobei die Erkrankung oder der pathologische Zustand ausgewählt ist aus endokrinen Störungen, Diabetes, Unfruchtbarkeit, Hormondefizienzen, Osteoporose, neurodegenerativen Störungen, Alzheimer'scher Erkrankung, Parkinson'scher Erkrankung, Huntington'scher Erkrankung, Herzkreislaufstörungen, Atherosklerose, Zuständen mit erhöhter Gerinnung, Zuständen mit verringerter Gerinnung, Herzerkrankung, zerebrovaskulären Ereignissen, metabolischen Störungen, Fettleibigkeit, Vitamindefizienzen, hämatologischen Störungen und neoplastischer Erkrankung.

28. Verfahren zur Herstellung eines Durchdringungsmoduls wie in Anspruch 1 beansprucht, wobei das Verfahren umfasst:
a) Transfizieren einer Herstellungszelle mit einem Vektor umfassend ein Nukleinsäuremolekül eines Fusionsproteins, welches das Durchdringungspeptid und einen Effektor kodiert, der operativ mit einer Expressionskontrollsequenz verknüpft ist;
b) Kultivieren der Herstellungszelle unter Bedingungen, welche die Herstellung eines Fusionsproteins erlauben, das aus dem Durchdringungspeptid und einem Effektorpeptid besteht; und
c) Isolieren des Fusionsproteins.

29. Durchdringungsmodul wie in Anspruch 1 beansprucht, wobei das Durchdringungspeptid mit einem Molekulargefäß fusioniert ist, wobei das Molekulargefäß einen Effektor einschließt.

30. Durchdringungsmodul wie in Anspruch 29 beansprucht, wobei der Effektor ausgewählt ist aus einem bioaktiven Peptid und einem pharmazeutisch aktiven Mittel.

31. Durchdringungsmodul wie in Anspruch 30 beansprucht, wobei das bioaktive Peptid ausgewählt ist aus Insulin, Wachtumshormon, einem Gonadotropin, einem Wachstumsfaktor, Erythropoietin, GM-CSF, α-MSH, Enkephalin, Dalargin, Kyotorphin, EPO, bFGF, Hirulog, einem LHRH Analog und neurotrophen Faktoren.

32. Durchdringungsmodul wie in Anspruch 31 beansprucht, wobei das pharmazeutisch aktive Mittel ausgewählt ist aus einem Antikoagulans, einem Toxin, einem Antibiotikum, einem antipathogenen Mittel, einem Antigen, einem Antikörperfragment, einem Vitamin, einem Immunmodulator, einem Enzym, einem antineoplastischen Mittel, Heparin, Methotraxat und einem therapeutischen Mittel.

33. Durchdringungsmodul wie in Anspruch 29 beansprucht, wobei das Molekulargefäß ausgewählt ist aus einem löslichen Rezeptor, einem Minirezeptor und einem Bindungsprotein.

34. Durchdringungsmodul wie in Anspruch 33 beansprucht, wobei der lösliche Rezeptor ein löslicher Insulinrezeptor ist, oder wobei der Minirezeptor eine ligandenbindende Domäne des Insulinrezeptors ist, oder wobei das Bindungsprotein der intrinsische Faktor ist.

35. Durchdringungsmodul wie in Anspruch 33 beansprucht, wobei der Effektor Vitamin B12 ist, oder ein Durchdringungsmodul wie in Anspruch 34 beansprucht, wobei der Effektor Insulin ist.

36. Pharmazeutische Zusammensetzung umfassend eine therapeutisch oder prophylaktisch wirksame Menge eines Durchdringungsmoduls wie in Anspruch 29 beansprucht, und einen pharmazeutisch verträglichen Träger.

37. Verfahren zur Herstellung eines Durchdringungsmoduls wie in Anspruch 1 beansprucht, wobei das Verfahren Verwenden von Festphasensynthese, um das Peptid zu konstruieren, und Fusionieren des Effektors an das Peptid, umfasst.

38. Durchdringungsmodul wie in Anspruch 1 beansprucht, das weiterhin einen oder mehrere Polyethylenglykol Rest/e, angeheftet an das Durchdringungspeptid, umfasst.

39. Verfahren wie in Anspruch 28 beansprucht, wobei das Fusionsprotein weiterhin einen oder mehrere Polyethylenglykol Rest/e, angeheftet an das Fusionsprotein, umfasst.

## Revendications

1. Module de pénétration comprenant un peptide pénétrant et un effecteur qui est couplé ou fusionné audit peptide pénétrant, ledit peptide pénétrant étant constitué d'au moins une séquence d'acides aminés choisie parmi
(a) SEQ ID NOS: 1 à 15; et
(b) SEQ ID NOS: 24 à 29;
lequel peptide pénétrant est capable d'une translocation au travers d'une barrière biologique.

2. Module de pénétration selon la revendication 1, dans lequel la séquence d'acides aminés est SEQ ID NO: 3, SEQ ID NO: 8, SEQ ID NO: 9, SEQ ID NO: 12 ou SEQ ID NO: 24.

3. Module de pénétration selon la revendication 1, dans lequel ledit effecteur est un peptide bioactif, choisi dans le groupe constitué par l'insuline, une hormone de croissance, une gonadotropine, un facteur de croissance, l'érythropoïétine, le facteur stimulant les colonies de granulocytes/monocytes (GM-CSF), l'αMSH, l'enképhaline, le dalargine, la kyotorphine, l'EPO, le bFGF, l'hirulogue, des analogues d'une hormone de libération de l'hormone lutéinisante (LH-RH) et des facteurs neurotrophiques.

4. Module de pénétration selon la revendication 1, dans lequel ledit effecteur est un agent actif d'un point de vue pharmaceutique, choisi dans le groupe constitué par un anticoagulant, une toxine, un antibiotique, un agent antipathogène, un antigène, un fragment d'anticorps, un immunomodulateur, une vitamine, une enzyme, un agent antinéoplasique et un agent thérapeutique.

5. Procédé *ex vivo* de translocation d'un peptide pénétrant au travers d'une barrière biologique, dans lequel le peptide pénétrant est un peptide pénétrant selon la revendication 1 ou la revendication 2.

6. Module de pénétration selon la revendication 1, dans lequel la translocation au travers d'une barrière biologique a lieu dans un tissu choisi parmi des cellules épithéliales et des cellules endothéliales.

7. Module de pénétration selon la revendication 1, dans lequel ladite barrière biologique est choisie parmi des jonctions occlusives et la membrane plasmatique, et dans lequel lesdites jonctions occlusives sont choisies parmi l'épithélium muqueux, l'épithélium intestinal, l'épithélium respiratoire et l'épithélium vasculaire.

8. Module de pénétration selon la revendication 7, dans lequel l'épithélium vasculaire comprend la barrière hémato-encéphalique.

9. Composition pharmaceutique comprenant une quantité efficace d'un point de vue thérapeutique ou prophylactique d'un module de pénétration selon la revendication 1, et un support acceptable d'un point de vue pharmaceutique.

10. Composition pharmaceutique selon la revendication 9, laquelle composition comprend en outre un mélange d'au moins deux substances choisies parmi un détergent non ionique, un détergent ionique, un inhibiteur de protéase et un agent réducteur.

11. Composition pharmaceutique selon la revendication 10, dans laquelle le détergent non ionique est un poloxamère, éventuellement le pluronic^{®} F-68.

12. Composition pharmaceutique selon la,revendication 10, dans laquelle le détergent ionique est un sel biliaire, éventuellement le taurodésoxycholate.

13. Composition pharmaceutique selon la revendication 10, dans laquelle l'inhibiteur de protéase est choisi parmi l'aprotinine et l'inhibiteur de trypsine du soja.

14. Composition pharmaceutique selon la revendication 10, dans laquelle l'agent réducteur est la N-acétyl-L-cystéine (NAC).

15. Procédé de production d'un module de pénétration selon la revendication 1, comprenant le couplage dudit effecteur audit peptide pénétrant.

16. Procédé selon la revendication 15, dans lequel le couplage dudit effecteur est réalisé par une liaison covalente.

17. Procédé selon la revendication 16, dans lequel ladite liaison covalente est une liaison peptidique.

18. Procédé selon la revendication 16, dans lequel la liaison covalente est réalisée par un réactif de pontage homo ou hétérofonctionnel, et dans lequel le réactif de pontage est éventuellement un réactif de type (N-maléimidométhyl)cyclohexane-1-carboxylate de succinimidyle (SMCC).

19. Procédé selon la revendication 16, dans lequel la liaison covalente est réalisée par un lieur peptidique, qui a éventuellement la séquence SEQ ID NO: 16 ou SEQ ID NO: 17, ou qui peut éventuellement être clivé par une enzyme.

20. Procédé selon la revendication 19, dans lequel le lieur peptidique est conçu pour être clivé par une enzyme activée de manière conditionnelle dans un certain état physiologique et dans lequel l'effecteur libéré influence de manière favorable ledit état physiologique.

21. Procédé selon la revendication 15, dans lequel le couplage dudit effecteur est réalisé par une liaison non covalente.

22. Procédé selon la revendication 21, dans lequel la liaison non covalente est réalisée par une fixation d'un fragment hydrophobe au peptide pénétrant, dans lequel le fragment hydrophobe permet au module de pénétration d'être incorporé à l'interface d'une vésicule hydrophobe dans laquelle l'effecteur est contenu.

23. Procédé selon la revendication 21, dans lequel la liaison non covalente est le résultat d'une interaction biotine-avidine ou biotine-streptavidine.

24. Procédé de translocation *ex vivo* d'un effecteur au travers d'une barrière biologique, ledit procédé comprenant :
a) le couplage dudit effecteur à un peptide pénétrant pour former un module de pénétration selon la revendication 1 ; et
b) l'introduction dudit module de pénétration au travers de la barrière biologique.

25. Utilisation d'un module de pénétration selon la revendication 1 dans la fabrication d'un vaccin pour une vaccination par voie orale, pour une administration à un sujet, le peptide pénétrant étant couplé à un antigène souhaitable.

26. Utilisation d'une composition pharmaceutique selon la revendication 9 dans la fabrication d'un médicament pour une administration à un sujet pour traiter ou prévenir une maladie ou un état pathologique, la composition pharmaceutique étant utilisée en une quantité suffisante pour traiter ou prévenir ladite maladie ou ledit état pathologique chez ledit sujet.

27. Utilisation selon la revendication 26, dans laquelle ladite maladie ou ledit état pathologique est choisi(e) parmi des troubles endocrines, un diabète, une infertilité, des déficiences hormonales, une ostéoporose, des troubles neurodégénératifs; une maladie d'Alzheimer, une maladie de Parkinson, une maladie de Huntington, des troubles cardiovasculaires, une athérosclérose, des états d'hypercoagulation, des états d'hypocoagulation, une maladie coronarienne, des événements cérébrovasculaires, des troubles métaboliques, une obésité, des déficiences en vitamine(s), des troubles hématologiques et une maladie néoplasique.

28. Procédé pour la production d'un module de pénétration selon la revendication 1, ledit procédé comprenant :
a) la transfection d'une cellule de production avec un vecteur comprenant une molécule d'acide nucléique d'une protéine fusionnée codant ledit peptide pénétrant et un effecteur lié de manière fonctionnelle à une séquence de contrôle de l'expression ;
b) la culture de ladite cellule de production dans des conditions qui permettent la production d'une protéine de fusion constituée du peptide pénétrant et d'un peptide effecteur ; et
c) l'isolement de ladite protéine fusionnée.

29. Module de pénétration selon la revendication 1, dans lequel ledit peptide pénétrant est fusionné à un vaisseau moléculaire, lequel vaisseau moléculaire contient un effecteur.

30. Module de pénétration selon la revendication 29, dans lequel l'effecteur est choisi entre un peptide bioactif et un agent actif d'un point de vue pharmaceutique.

31. Module de pénétration selon la revendication 30, dans lequel ledit peptide bioactif est choisi parmi l'insuline, une hormone de croissance, une gonadotropine, un facteur de croissance, l'érythropoïétine, le GM-CSF, l'αMSH, l'enképhaline, la dalargine, la kyotorphine, l'EPO, le bFGF, l'hirulogue, un analogue de LH-RH et des facteurs neurotrophiques.

32. Module de pénétration selon la revendication 31, dans lequel ledit agent actif d'un point de vue pharmaceutique est choisi parmi un anticoagulant, une toxine, un antibiotique, un agent antipathogène, un antigène, un fragment d'anticorps, une vitamine, un immunomodulateur, une enzyme, un agent antinéoplasique, l'héparine, le méthotréxate et un agent thérapeutique.

33. Module de pénétration selon la revendication 29, dans lequel le vaisseau moléculaire est choisi parmi un récepteur soluble, un minirécepteur et une protéine de liaison.

34. Module de pénétration selon la revendication 33, dans lequel le récepteur soluble est un récepteur de l'insuline soluble, ou dans lequel le minirécepteur est le domaine de liaison à un ligand du récepteur de l'insuline, ou dans lequel la protéine de liaison est un facteur intrinsèque.

35. Module de pénétration selon la revendication 33, dans lequel l'effecteur est la vitamine B12 ou module de pénétration selon la revendication 34, dans lequel l'effecteur est l'insuline.

36. Composition pharmaceutique comprenant une quantité efficace d'un point de vue thérapeutique ou prophylactique d'un module de pénétration selon la revendication 29 et un support acceptable d'un point de vue pharmaceutique.

37. Procédé de production d'un module de pénétration selon la revendication 1, ledit procédé comprenant l'utilisation d'une synthèse en phase solide pour construire le peptide et fusionner ledit effecteur au peptide.

38. Module de pénétration selon la revendication 1, comprenant en outre un ou plusieurs résidus de polyéthylèneglycol fixés au peptide pénétrant.

39. Procédé selon la revendication 28, dans lequel la protéine fusionnée comprend en outre un ou plusieurs résidus de polyéthylèneglycol fixés à la protéine fusionnée.


### SEQUENCE LISTING

<110> Yissum Research Development Company Ben-Sasson, Shmuel A Cohen, Einat
<120> Amino Acid sequences capable of Facilitating Penetration Across a Biological Barrier
<130> 24348-501-061
<140> PCT/IB03/00968
   <141> 2003-02-07
<150> 60/355,396
   <151> 2002-02-07
<160> 59
<170> PatentIn ver. 2.1
<210> 1
   <211> 23
   <212> PRT
   <213> Haemophilus influenzae
<400> 1
   Asn Tyr His Asp Ile Val Leu Ala Leu Ala Gly Val Cys Gln Ser Ala
    1               5                  10                  15
   Lys Leu Val His Gln Leu Ala
               20
<210> 2
   <211> 23
   <212> PRT
   <213> Pasteurella multocida
<400> 2
   Asn Tyr Tyr Asp Ile Thr Leu Ala Leu Ala Gly Val Cys Gln Ala Ala
    1               5                  10                  15
   Lys Leu Val Gln Gln Phe Ala
               20
<210> 3
   <211> 23
   <212> PRT
   <213> Escherichia coli
<400> 3
   Asn Tyr Tyr Asp Ile Thr Leu Ala Leu Ala Gly Ile Cys Gln Ser Ala
   1 5 10 15
   Arg Leu Val Gln Gln Leu Ala
   20
<210> 4
   <211> 23
   <212> PRT
   <213> vibrio cholerae
<400> 4
<210> 5
   <211> 23
   <212> PRT
   <213> Buchnera aphidicola
<400> 5
   Lys Ile His Leu Ile Thr Leu Ser Leu Ala Gly Ile Cys Gln Ser Ala
   1 5 10 15
   His Leu Val Gln Gln Leu Ala
   20
<210> 6
   <211> 23
   <212> PRT
   <213> Pseudomonas aeruginosa
<400> 6
   Asp Pro Arg Gln Gln Leu Ile Ala Leu Gly Ala Val Phe Glu Ser Ala
   1 5 10 15
   Ala Leu Val Asp Lys Leu Ala
   20
<210> 7
   <211> 23
   <212> PRT
   <213> Xylella fastidiosa
<400> 7
   Leu Ile Asp Asn Arg Val Leu Ala Leu Ala Gly Val Val Gln Ala Leu
   1 5 10 15
   Gln Gln Val Arg Gln Ile Ala
   20
<210> 8
   <211> 23
   <212> PRT
   <213> Rhizobium loti
<400> 8
   Asn Leu Pro Pro Ile Val Leu Ala Val Ile Gly Ile Cys Ala Ala Val
   1 5 10 15
   Phe Leu Leu Gln Gln Tyr Val
   20
<210> 9
   <211> 23
   <212> PRT
   <213> Homo sapiens
<400> 9
   Asn Tyr Phe Ile Val Asn Leu Ala Leu Ala Asp Leu Cys Met Ala Ala
   1 5 10 15
   Phe Asn Ala Ala Phe Asn Phe
   20
<210> 10
   <211> 23
   <212> PRT
   <213> Chlamydia pneumoniae
<400> 10
   Thr Ala Phe Asp Phe Asn Lys Met Leu Asp Gly Val Cys Thr Tyr Val
   1 5 10 15
   Lys Gly Val Gln Gln Tyr Leu
   20
<210> 11
   <211> 23
   <212> PRT
   <213> Rhizobium loti
<400> 11
   Arg Ala Ile Leu Ile Pro Leu Ala Leu Ala Gly Leu Cys Gln Val Ala
   1 5 10 15
   Arg Ala Gly Asp Ile Ser Ser
   20
<210> 12
   <211> 25
   <212> PRT
   <213> Bacillus subtilis
<400> 12
   Met Arg Asn Leu Thr Lys Thr Ser Leu Leu Leu Ala Gly Leu Cys Thr
   1 5 10 15
   Ala Ala Gln Met Val Phe Val Thr His
   20 25
<210> 13
   <211> 25
   <212> PRT
   <213> Kingella denitrificans
<400> 13
   Ile Glu Leu Met Ile Val Ile Ala Ile Ile Gly Ile Leu Ala Ala Ile
   1 5 10 15
   Ala Leu Pro Ala Tyr Gln Glu Tyr Val
   20 25
<210> 14
   <211> 25
   <212> PRT
   <213> Eikenella corrodens
<400> 14
   Ile Glu Leu Met Ile Val Ile Ala Ile Ile Gly Ile Leu Ala Ala Ile
   1 5 10 15
   Ala Leu Pro Ala Tyr Gln Asp Tyr Val
   20 25
<210> 15
   <211> 16
   <212> PRT
   <213> Artificial Sequence
<220>
   <223> Description of Artificial Sequence: Zonula occludens toxin
<400> 15
   Ala Ser Phe Gly Phe Cys Ile Gly Arg Leu Cys Val Gln Asp Gly Phe
   1 5 10 15
<210> 16
   <211> 4
   <212> PRT
   <213> Artificial sequence
<220>
   <223> Description of Artificial Sequence: Cleavable linker peptide
<400> 16
   Ile Glu Gly Arg
   1
<210> 17
   <211> 6
   <212> PRT
   <213> Artificial sequence
<220>
   <223> Description of Artificial Sequence: cleavable linker sequence
<400> 17
   Gly Gly Lys Gly Gly Lys
   1 5
<210> 18
   <211> 30
   <212> PRT
   <213> Artificial sequence
<220>
   <223> Description of Artificial sequence: Penetrating peptide/recombinant insulin chimera
<220>
   <221> VARIANT
   <222> (30)
   <223> wherein Xaa is a recombinant insulin polypeptide.
<400> 18
   Asn Tyr Tyr Asp Ile Thr Leu Ala Leu Ala Gly Ile Cys Gln Ser Ala
   1 5 10 15
   Arg Leu Val Gln Gln Leu Ala Gly Gly Ile Glu Gly Arg Xaa
   20 25 30
<210> 19
   <211> 26
   <212> PRT
   <213> Artificial sequence
<220>
   <223> Description of Artificial Sequence: penetrating peptide/recombinant insulin chimera
<220>
   <221> VARIANT
   <222> (26)
   <223> wherein xaa is a recombinant insulin polypeptide.
<400> 19
   Asn Tyr Tyr Asp Ile Thr Leu Ala Leu Ala Gly Ile Cys Gln Ser Ala
   1 5 10 15
   Arg Leu Val Gln Gln Leu Ala Gly Gly Xaa
   20 25
<210> 20
   <211> 31
   <212> PRT
   <213> Artificial sequence
<220>
   <223> Description of Artificial sequence: Penetrating Peptide/Heparin chimera
<220>
   <221> VARIANT
   <222> (31)
   <223> wherein xaa is a heparin polypeptide.
<400> 20
   Asn Tyr Tyr Asp Ile Thr Leu Ala Leu Ala Gly Ile Cys Gln Ser Ala
   1 5 10 15
   Arg Leu Val Gln Gln Leu Ala Gly Gly Ile Glu Gly Arg Lys Xaa
   20 25 30
<210> 21
   <211> 27
   <212> PRT
   <213> Artificial Sequence
<220>
   <223> Description of Artificial sequence: Penetrating Protein/Heparin Chimera
<220>
   <221> VARIANT
   <222> (27)
   <223> wherein Xaa is a heparin polypeptide.
<400> 21
   Asn Tyr Tyr Asp Ile Thr Leu Ala Leu Ala Gly Ile Cys Gln Ser Ala
   1               5                   10                  15
   Arg Leu Val Gln Gln Leu Ala Gly Gly Lys Xaa
               20                  25
<210> 22
   <211> 32
   <212> PRT
   <213> Artificial Sequence
<220>
   <223> Description of Artificial Sequence: Penetrating peptide
<220>
   <221> VARIANT
   <222> (1)
   <223> wherein Xaa is an acetyl group.
<220>
   <221> VARIANT
   <222> (32)
   <223> wherein Xaa is a free amino group.
<400> 22
   Xaa Asn Tyr Tyr Asp Ile Thr Leu Ala Leu Ala Gly Ile Cys Gln Ser
   1               5                   10                  15
   Ala Arg Leu Val Gln Gln Leu Ala Gly Gly Gly Lys Gly Gly Lys Xaa
               20                  25                  30
<210> 23
   <211> 26
   <212> PRT
   <213> Artificial Sequence
<220>
   <223> Description of Artificial Sequence: Penetrating Protein/Insulin Receptor chimera
<220>
   <221> VARIANT
   <222> (26)
   <223> wherein Xaa is a linearized insulin receptor sequence.
<400> 23
   Asn Tyr Tyr Asp Ile Thr Leu Ala Leu Ala Gly Ile Cys Gln Ser Ala
   1 5 10 15
   Arg Leu Val Gln Gln Leu Ala Gly Gly Xaa
   20 25
<210> 24
   <211> 23
   <212> PRT
   <213> Homo sapiens
<400> 24
   Asn Tyr Phe Leu Val Asn Leu Ala Phe Ala Glu Ala Ser Met Ala Ala
   1 5 10 15
   Phe Asn Thr Val Val Asn Phe
   20
<210> 25
   <211> 24
   <212> PRT
   <213> Escherichia coli
<400> 25
   Met Asn Tyr Tyr Asp Ile Thr Leu Ala Leu Ala Gly Ile Cys Gln Ser
   1 5 10 15
   Ala Arg Leu Val Gln Gln Leu Ala
   20
<210> 26
   <211> 23
   <212> PRT
   <213> Escherichia coli
<400> 26
   Met Tyr Tyr Asp Ile Thr Leu Ala Leu Ala Gly Ile Cys Gln Ser Ala
   1 5 10 15
   Arg Leu Val Gln Gln Leu Ala
   20
<210> 27
   <211> 22
   <212> PRT
   <213> Escherichia coli
<400> 27
   Met Tyr Asp Ile Thr Leu Ala Leu Ala Gly Ile Cys Gln Ser Ala Arg
   1 5 10 15
   Leu Val Gln Gln Leu Ala
   20
<210> 28
   <211> 23
   <212> PRT
   <213> Bacillus subtilis
<400> 28
   Met Arg Asn Leu Thr Arg Thr Ser Leu Leu Leu Ala Gly Leu Cys Thr
   1 5 10 15
   Ala Ala Gln Met Val Phe Val
   20
<210> 29
   <211> 23
   <212> PRT
   <213> Haemophilus influenzae
<400> 29
   Asn Tyr His Asp Ile Val Leu Ala Leu Ala Gly Val Cys Gln Ser Ala
   1 5 10 15
   Arg Leu Val His Gln Leu Ala
   20
<210> 30
   <211> 32
   <212> PRT
   <213> Artificial Sequence
<220>
   <223> Description of Artificial sequence: Penetrating Peptide
<220>
   <221> VARIANT
   <222> (1)
   <223> wherein Xaa is an acetyl group.
<220>
   <221> VARIANT
   <222> (32)
   <223> wherein Xaa is a free amino group.
<400> 30
   Xaa Asn Leu Pro Pro Ile Val Leu Ala Val Ile Gly Ile Cys Ala Ala
   1 5 10 15
   Val Phe Leu Leu Gln Gln Tyr Val Gly Gly Gly Lys Gly Gly Lys Xaa
   20 25 30
<210> 31
   <211> 32
   <212> PRT
   <213> Artificial sequence
<220>
   <223> Description of Artificial Sequence: Penetrating Peptide
<220>
   <221> VARIANT
   <222> (1)
   <223> wherein Xaa is an acetyl group.
<220>
   <221> VARIANT
   <222> (32)
   <223> wherein Xaa is a free amino group.
<400> 31
   Xaa Asn Tyr Phe Ile Val Asn Leu Ala Leu Ala Asp Leu Cys Met Ala
   1 5 10 15
   Ala Phe Asn Ala Ala Phe Asn Phe Gly Gly Gly Lys Gly Gly Lys Xaa
   20 25 30
<210> 32
   <211> 32
   <212> PRT
   <213> Artificial sequence
<220>
   <223> Description of Artificial Sequence: Penetrating Peptide
<220>
   <221> VARIANT
   <222> (1)
   <223> wherein xaa is an acetyl group.
<220>
   <221> VARIANT
   <222> (32)
   <223> wheein xaa is a free amino group.
<400> 32
   Xaa Met Arg Asn Leu Thr Arg Thr Ser Leu Leu Leu Ala Gly Leu Cys
   1 5 10 15
   Thr Ala Ala Gln Met Val Phe Val Gly Gly Gly Lys Gly Gly Lys Xaa
   20 25 30
<210> 33
   <211> 31
   <212> PRT
   <213> Artificial Sequence
<220>
   <223> Description of Artificial sequence: Penetrating Peptide
<220>
   <221> VARIANT
   <222> (1)
   <223> wherein xaa is an acetyl group.
<220>
   <221> VARIANT
   <222> (31)
   <223> wherein Xaa is a free amino group.
<400> 33
   Xaa Asn Tyr His Asp Ile Val Leu Ala Leu Ala Gly Val Cys Gln Ser
   1 5 10 15
   Ala Arg Leu Val His Gln Leu Ala Gly Gly Lys Gly Gly Lys Xaa
   20 25 30
<210> 34
   <211> 31
   <212> PRT
   <213> Artificial sequence
<220>
   <223> Description of Artificial Sequence: Penetrating Peptide
<220>
   <221> VARIANT
   <222> (1)
   <223> wherein xaa is an acetyl group.
<220>
   <221> VARIANT
   <222> (31)
   <223> wherein xaa is a free amino group.
<400> 34
   Xaa Asn Tyr Phe Leu Val Asn Leu Ala Phe Ala Glu Ala Ser Met Ala
   1 5 10 15
   Ala Phe Asn Thr Val Val Asn Phe Gly Gly Lys Gly Gly Lys Xaa
   20 25 30
<210> 35
   <211> 33
   <212> PRT
   <213> Artificial Sequence
<220>
   <223> Description of Artificial sequence: Penetrating Peptide
<220>
   <221> VARIANT
   <222> (1)
   <223> wherein Xaa is an acetyl group.
<220>
   <221> VARIANT
   <222> (33)
   <223> wherein xaa is a free amino group.
<400> 35
   Xaa Met Asn Tyr Tyr Asp Ile Thr Leu Ala Leu Ala Gly Ile Cys Gln
   1 5 10 15
   Ser Ala Arg Leu Val Gln Gln Leu Ala Gly Gly Gly Lys Gly Gly Lys
   20 25 30
   Xaa
<210> 36
   <211> 32
   <212> PRT
   <213> Artificial sequence
<220>
   <223> Description of Artificial sequence: Penetrating Peptide
<220>
   <221> VARIANT
   <222> (1)
   <223> wherein Xaa is an acetyl group.
<220>
   <221> VARIANT
   <222> (32)
   <223> wherein Xaa is a free amino group.
<400> 36
   Xaa Met Tyr Tyr Asp Ile Thr Leu Ala Leu Ala Gly Ile Cys Gln Ser
   1 5 10 15
   Ala Arg Leu Val Gln Gln Leu Ala Gly Gly Gly Lys Gly Gly Lys Xaa
   20 25 30
<210> 37
   <211> 31
   <212> PRT
   <213> Artificial sequence
<220>
   <223> Description of Artificial sequence: Penetrating Peptide
<220>
   <221> VARIANT
   <222> (1)
   <223> wherein Xaa is an acetyl group.
<220>
   <221> VARIANT
   <222> (31)
   <223> wherein xaa is a free amino group.
<400> 37
   Xaa Met Tyr Asp Ile Thr Leu Ala Leu Ala Gly Ile Cys Gln Ser Ala
   1 5 10 15
   Arg Leu Val Gln Gln Leu Ala Gly Gly Gly Lys Gly Gly Lys Xaa
   20 25 30
<210> 38
   <211> 16
   <212> PRT
   <213> Artificial Sequence
<220>
   <223> Description of Artificial Sequence: Penetrating Protein consensus sequence
<220>
   <221> VARIANT
   <222> (1)
   <223> wherein xaa is an Ala, Val, Leu, Ile, Met, Phe, or Pro.
<220>
   <221> VARIANT
   <222> (2)
   <223> wherein xaa is any amino acid.
<220>
   <221> VARIANT
   <222> (3)
   <223> wherein Xaa is an Ala, val, Leu, Ile, Met, Phe, or Pro.
<220>
   <221> VARIANT
   <222> (4)
   <223> wherein xaa is any amino acid.
<220>
   **<221> VARIANT**
   <222> (5)
   <223> wherein xaa is an Ala, Val, Leu, Ile, Met, Phe, or Pro.
<220>
   <221> VARIANT
   <222> (6)
   <223> wherein xaa is any amino acid.
<220>
   <221> VARIANT
   <222> (7)
   <223> wherein Xaa is an Ala, val, Leu, Ile, Met, Phe, or Pro.
<220>
   <221> VARIANT
   <222> (8)
   <223> Wherein Xaa is any amino acid.
<220>
   <221> VARIANT
   <222> (9)
   <223> wherein Xaa is Lys, Arg, Asp, or Glu.
<220>
   <221> VARIANT
   <222> (10)
   <223> Wherein Xaa is an Ala, Val, Leu, Ile, Met, Phe, or Pro.
<220>
   <221> VARIANT
   <222> (11)
   <223> wherein xaa is any amino acid.
<220>
   <221> VARIANT
   <222> (12)
   <223> Wherein Xaa is an Ala, Val, Leu, Ile, Met, Phe, or Pro.
<220>
   <221> VARIANT
   <222> (13)
   <223> wherein Xaa is any amino acid.
<220>
   <221> VARIANT
   <222> (14)
   <223> wherein Xaa is a Lys, Arg, Asp, or Glu.
<220>
   <221> VARIANT
   <222> (15)
   <223> wherein Xaa is any amino acid.
<220>
   <221> VARIANT
   <222> (16)
   <223> Wherein Xaa is an Ala, Val, Leu, Ile, Met, Phe, or Pro.
<400> 38
   Xaa Xaa Xaa Xaa Xaa Xaa Xaa Xaa Xaa Xaa Xaa Xaa Xaa Xaa Xaa Xaa
   1 5 10 15
<210> 39
   <211> 23
   <212> PRT
   <213> Artificial Sequence
<220>
   <223> Description of Artificial sequence: Penetrating Protein Consensus sequence
<220>
   <221> VARIANT
   <222> (1)
   <223> wherein xaa is Lys, Arg, Asp, or Glu.
<220>
   <221> VARIANT
   <222> (2)
   <223> Wherein Xaa is an Ala, val, Leu, Ile, Met, Phe, or Pro.
<220>
   <221> VARIANT
   <222> (3)
   <223> wherein Xaa is any amino acid.
<220>
   <221> VARIANT
   <222> (4)..(5)
   <223> Wherein Xaa is an Ala, val, Leu, Ile, Met, Phe, or Pro.
<220>
   <221> VARIANT
   <222> (6)
   <223> wherein Xaa is any amino acid.
<220>
   <221> VARIANT
   <222> (7)
   <223> wherein Xaa is an Ala, val, Leu, Ile, Met, Phe, or Pro.
<220>
   <221> VARIANT
   <222> (8)
   <223> wherein Xaa is any amino acid.
<220>
   <221> VARIANT
   <222> (9)..(10)
   <223> Wherein Xaa is an Ala, val, Leu, Ile, Met, Phe, or Pro.
<220>
   <221> VARIANT
   <222> (11)
   <223> wherein xaa is any amino acid.
<220>
   <221> VARIANT
   <222> (12)
   <223> Wherein Xaa is an Ala, Val, Leu, Ile, Met, Phe, or Pro.
<220>
   <221> VARIANT
   <222> (13)..(15)
   <223> Wherein Xaa is any amino acid.
<220>
   <221> VARIANT
   <222> (16)
   <223> wherein xaa is an Ala, val. Leu, Ile, Met, Phe, or Pro.
<220>
   <221> VARIANT
   <222> (17)
   <223> wherein Xaa is any amino acid.
<220>
   <221> VARIANT
   <222> (18)..(19)
   <223> wherein Xaa is an Ala, val, Leu, Ile, Met, Phe, or Pro.
<220>
   <221> VARIANT
   <222> (20)..(21)
   <223> wherein Xaa is any amino acid.
<220>
   <221> VARIANT
   <222> (22)..(23)
   <223> wherein Xaa is an Ala, Val, Leu, Ile, Met, Phe, or Pro.
<400> 39
   Xaa Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Xaa Xaa
               20
<210> 40
   <211> 23
   <212> PRT
   <213> Artificial sequence
<220>
   <223> Description of Artificial Sequence: Penetrating Protein Consensus Sequence
<220>
   <221> VARIANT
   <222> (1)
   <223> wherein Xaa is Lys, Arg, Asp, or Glu.
<220>
   <221> VARIANT
   <222> (2)
   <223> wherein xaa is an Ala, val, Leu, Ile, Met, Phe, or Pro.
<220>
   <221> VARIANT
   <222> (3)
   <223> wherein Xaa is Lys, Arg, Asp, or Glu.
<220>
   <221> VARIANT
   <222> (4)..(5)
   <223> wherein Xaa is any amino acid.
<220>
   <221> VARIANT
   <222> (6)..(9)
   <223> wherein Xaa is an Ala, val, Leu, Ile, Met, Phe, or Pro.
<220>
   <221> VARIANT
   <222> (10)
   <223> wherein xaa is any amino acid.
<220>
   <221> VARIANT
   <222> (11)..(13)
   <223> wherein Xaa is an Ala, val, Leu, Ile, Met, Phe, or Pro.
<220>
   <221> VARIANT
   <222> (14)
   <223> wherein xaa is Lys, Arg, Asp, or Gu.
<220>
   <221> VARIANT
   <222> (15)
   <223> wherein xaa is any amino acid.
<220>
   <221> VARIANT
   <222> (16)..(19)
   <223> wherein Xaa is n Ala, val, Leu, Ile, Met, Phe, or Pro.
<220>
   <221> VARIANT
   <222> (20)..(21)
   <223> wherein Xaa is Lys, Arg, Asp, or Glu.
<220>
   <221> VARIANT
   <222> (22)..(23)
   <223> wherein Xaa is an Ala, Val, Leu, Ile, Met, Phe, or Pro.
<400> 40
   Xaa Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Xaa Xaa
               20
<210> 41
   <211> 23
   <212> PRT
   <213> Artificial Sequence
<220>
   <223> Description of Artificial Sequence: Penetrating Protein consensus sequence
<220>
   <221> VARIANT
   <222> (1)
   <223> Wherein Xaa is Lys, Arg, Asp, or Glu.
<220>
   <221> VARIANT
   <222> (2)..(10)
   <223> wherein Xaa is an Ala, val, Leu, Ile, Met, Phe, or Pro.
<220>
   <221> VARIANT
   <222> (11)
   <223> wherein xaa is any amino acid.
<220>
   <221> VARIANT
   <222> (12)
   <223> wherein Xaa is an Ala, val. Leu, Ile, Met, Phe, or Pro.
<220>
   <221> VARIANT
   <222> (13)..(14)
   <223> wherein Xaa is any amino acid.
<220>
   <221> VARIANT
   <222> (15)..(16)
   <223> wherein Xaa is an Ala, Val, Leu, Ile, Met, Phe, or Pro.
<220>
   <221> VARIANT
   <222> (17)
   <223> wherein Xaa is Lys, Arg, Asp, or Glu.
<220>
   <221> VARIANT
   <222> (18)
   <223> wherein Xaa is an Ala, val, Leu, Ile, Met, Phe, or Pro.
<220>
   <221> VARIANT
   <222> (19)
   <223> wherein xaa is any amino acid.
<220>
   <221> VARIANT
   <222> (20)
   <223> wherein Xaa is Lys, Arg, Asp, or Glu.
<220>
   <221> VARIANT
   <222> (21)
   <223> wherein xaa is an Ala, val, Leu, Ile, Met, Phe, or Pro.
<220>
   <221> VARIANT
   <222> (22)..(23)
   <223> wherein Xaa is any amino acid.
<400> 41
   Xaa Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Xaa Xaa
               20
<210> 42
   <211> 25
   <212> PRT
   <213> Artificial Sequence
<220>
   <223> Description of Artificial sequence: Penetrating Protein Consensus Sequence
<220>
   <221> VARIANT
   <222> (1)
   <223> wherein xaa is an Ala, val, Leu, Ile, Met, Phe, or Pro.
<220>
   <221> VARIANT
   <222> (2)
   <223> wherein xaa is Lys, Arg, Asp, or Glu.
<220>
   <221> VARIANT
   <222> (3)..(10)
   <223> wherein Xaa is an Ala, val, Leu, Ile, Met, Phe, or Pro.
<220>
   <221> VARIANT
   <222> (11)
   <223> Wherein Xaa is any amino acid.
<220>
   <221> VARIANT
   <222> (12)..(20)
   <223> wherein xaa is an Ala, Val, Leu, Ile, Met, Phe, or Pro.
<220>
   <221> VARIANT
   <222> (21)..(22)
   <223> wherein xaa is any amino acid.
<220>
   <221> VARIANT
   <222> (23)
   <223> wherein Xaa is Lys, Arg, Asp, or Glu.
<220>
   <221> VARIANT
   <222> (24)
   <223> wherein Xaa is any amino acid.
<220>
   <221> VARIANT
   <222> (25)
   <223> wherein Xaa is an Ala, val, Leu, Ile, Met, Phe, or Pro.
<400> 42
   Xaa Xaa Xaa Xaa Xaa Xaa Xaa Xaa Xaa Xaa Xaa Xaa Xaa Xaa Xaa Xaa
    1               5                   10                  15
   Xaa Xaa Xaa Xaa Xaa Xaa Xaa Xaa Xaa
                20                  25
<210> 43
   <211> 23
   <212> PRT
   <213> Artificial Sequence
<220>
   <223> Description of Artificial Sequence: Penetrating Protein Consensus sequence
<220>
   <221> VARIANT
   <222> (1)..(2)
   <223> Wherein Xaa is Ala, Val, Leu, Ile, Met, Phe, or Pro.
<220>
   <221> VARIANT
   <222> (3)
   <223> wherein Xaa is Lys, Arg, Asp, or Glu.
<220>
   <221> VARIANT
   <222> (4)
   <223> wherein xaa is any amino acid.
<220>
   <221> VARIANT
   <222> (5)
   <223> Wherein Xaa is Lys, Arg, Asp, or Glu.
<220>
   <221> VARIANT
   <222> (6)..(10)
   <223> Wherein Xaa is Ala, Val, Leu, Ile, Met, Phe, or Pro.
<220>
   <221> VARIANT
   <222> (11)
   <223> wherein xaa is any amino acid.
<220>
   <221> VARIANT
   <222> (12)..(13)
   <223> wherein xaa is Ala, val, Leu, Ile, Met, Phe, or Pro.
<220>
   <221> VARIANT
   <222> (14)
   <223> wherein Xaa is any amino acid.
<220>
   <221> VARIANT
   <222> (15)..(16)
   <223> wherein Xaa is Ala, val, Leu, Ile, Met, Phe, or Pro.
<220>
   <221> VARIANT
   <222> (17)..(18)
   <223> wherein Xaa is any amino acid.
<220>
   <221> VARIANT
   <222> (19)
   <223> Wherein Xaa is Ala, Val, Leu, Ile, Met, Phe, or Pro.
<220>
   <221> VARIANT
   <222> (20)
   <223> wherein xaa is Lys, Arg, Asp, or Glu.
<220>
   <221> VARIANT
   <222> (21)
   <223> wherein xaa is any amino acid.
<220>
   <221> VARIANT
   <222> (22)..(23)
   <223> wherein Xaa is Ala, val, Leu, Ile, Met, Phe, or Pro.
<400> 43
   Xaa Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Xaa Xaa
               20
<210> 44
   <211> 23
   <212> PRT
   <213> Artificial Sequence
<220>
   <223> Description of Artificial Sequence: Penetrating Protein Consensus sequence
<220>
   <221> VARIANT
   <222> (1)
   <223> wherein Xaa is any amino acid.
<220>
   <221> VARIANT
   <222> (2)..(10)
   <223> wherein Xaa is Ala, val, Leu, Ile, Met, Phe, or Pro.
<220>
   <221> VARIANT
   <222> (11)
   <223> wherein Xaa is any amino acid.
<220>
   <221> VARIANT
   <222> (12)
   <223> wherein Xaa is Ala, val, Leu, Ile, Met, Phe, or Pro.
<220>
   <221> VARIANT
   <222> (13)
   <223> wherein xaa is any amino acid.
<220>
   <221> VARIANT
   <222> (14)..(19)
   <223> wherein Xaa is Ala, Val, Leu, Ile, Met, Phe, or Pro.
<220>
   <221> VARIANT
   <222> (20)..(22)
   <223> wherein Xaa is any amino acid.
<220>
   <221> VARIANT
   <222> (23)
   <223> wherein Xaa is Ala, Val, Leu, Ile, Met, Phe, or Pro.
<400> 44
   Xaa Xaa Xaa Xaa Xaa Xaa Xaa Xaa Xaa Xaa Xaa Xaa Xaa Xaa Xaa Xaa
   1 5 10 15
   Xaa Xaa Xaa Xaa Xaa Xaa Xaa
   20
<210> 45
   <211> 23
   <212> PRT
   <213> Artificial Sequence
<220>
   <223> Description of Artificial Sequence: Penetrating Protein consensus Sequence
<220>
   <221> VARIANT
   <222> (1)..(2)
   <223> wherein Xaa is any amino acid.
<220>
   <221> VARIANT
   <222> (3)..(5)
   <223> wherein Xaa is Ala, val, Leu, Ile, Met, Phe, or Pro.
<220>
   <221> VARIANT
   <222> (6)
   <223> wherein xaa is any amino acid.
<220>
   <221> VARIANT
   <222> (7) .. (10)
   <223> wherein Xaa is Ala, val, Leu, Ile, Met, Phe, or Pro.
<220>
   <221> VARIANT
   <222> (11)
   <223> wherein Xaa is Lys, Arg, Asp, or Glu.
<220>
   <221> VARIANT
   <222> (12)
   <223> wherein Xaa is Ala, val, Leu, Ile, Met, Phe, or Pro.
<220>
   <221> VARIANT
   <222> (13)
   <223> wherein Xaa is any amino acid.
<220>
   <221> VARIANT
   <222> (14)..(17)
   <223> wherein Xaa is Ala, Val, Leu, Ile, Met, Phe, or Pro.
<220>
   <221> VARIANT
   <222> (18)
   <223> wherein Xaa is any amino acid.
<220>
   <221> VARIANT
   <222> (19)
   <223> Wherein Xaa is Ala, Val, Leu, Ile, Met, Phe, or Pro.
<220>
   <221> VARIANT
   <222> (20)..(21)
   <223> wherein Xaa is Ala, Val, Leu, Ile, Met, Phe, Pro, or nothing.
<220>
   <221> VARIANT
   <222> (22)
   <223> wherein xaa is any amino acid.
<220>
   <221> VARIANT
   <222> (23)
   <223> Wherein Xaa is Ala, Val, Leu, Ile, Met, Phe, or Pro.
<400> 45
   Xaa Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Xaa Xaa
               20
<210> 46
   <211> 24
   <212> PRT
   <213> Artificial Sequence
<220>
   <223> Description of Artificial sequence: Penetrating Protein Consensus Sequence
<220>
   <221> VARIANT
   <222> (1)
   <223> wherein Xaa is any amino acid.
<220>
   <221> VARIANT
   <222> (2)..(3)
   <223> wherein Xaa is Ala, val, Leu, Ile, Met, Phe, or Pro.
<220>
   <221> VARIANT
   <222> (4)
   <223> wherein Xaa is any ammo acid.
<220>
   <221> VARIANT
   <222> (5)
   <223> Wherein Xaa is Lys, Arg, Asp, or Glu.
<220>
   <221> VARIANT
   <222> (6)
   <223> wherein Xaa is Ala, val, Leu, Ile, Met, Phe, or Pro.
<220>
   <221> VARIANT
   <222> (7)
   <223> wherein Xaa is any amino acid.
<220>
   <221> VARIANT
   <222> (8)
   <223> wherein Xaa is Lys, Arg, Asp, or Glu.
<220>
   <221> VARIANT
   <222> (9)..(10)
   <223> wherein Xaa is Ala, Val, Leu, Ile, Met, Phe, or Pro.
<220>
   <221> VARIANT
   <222> (11)
   <223> Wherein Xaa is Arg, Lys, Asp, or Glu.
<220>
   <221> VARIANT
   <222> (12)
   <223> wherein Xaa is any amino acid.
<220>
   <221> VARIANT
   <222> (13)
   <223> wherein Xaa is Ala, Val, Leu, Ile, Met, Phe, or Pro.
<220>
   <221> VARIANT
   <222> (14)..(16)
   <223> wherein Xaa is any amino acid.
<220>
   <221> VARIANT
   <222> (17)
   <223> wherein Xaa is Ala, val, Leu, Ile, Met, Phe, or Pro.
<220>
   <221> VARIANT
   <222> (18)
   <223> wherein Xaa is Lys, Arg, Asp, or Glu.
<220>
   <221> VARIANT
   <222> (19)
   <223> wherein Xaa is any amino acid.
<220>
   <221> VARIANT
   <222> (20)
   <223> Wherein Xaa is Ala, val, Leu, Ile, Met, Phe, or Pro.
<220>
   <221> VARIANT
   <222> (21)..(23)
   <223> wherein Xaa is any amino acid.
<220>
   <221> VARIANT
   <222> (24)
   <223> wherein Xaa is Ala, Val, Leu, Ile, Met, Phe, or Pro.
<400> 46
   Xaa Xaa Xaa Xaa Xaa Xaa Xaa Xaa Xaa Xaa Xaa Xaa Xaa Xaa Xaa Xaa
   1 5 10 15
   Xaa Xaa Xaa Xaa Xaa Xaa Xaa Xaa
   20
<210> 47
   <211> 25
   <212> PRT
   <213> Artificial sequence
<220>
   <223> Description of Artificial Sequence: Penetrating Protein Consensus Sequence
<220>
   <221> VARIANT
   <222> (1)
   <223> wherein Xaa is Ala, val, Leu, Ile, Met, Phe, or Pro.
<220>
   <221> VARIANT
   <222> (2)
   <223> wherein Xaa is Lys, Arg, Asp, or Glu.
<220>
   <221> VARIANT
   <222> (3)
   <223> wherein Xaa is any amino acid.
<220>
   <221> VARIANT
   <222> (4)
   <223> wherein Xaa is Ala, val, Leu, Ile, Met, Phe, or Pro.
<220>
   <221> VARIANT
   <222> (5)
   <223> wherein Xaa is any amino acid.
<220>
   <221> VARIANT
   <222> (6)
   <223> wherein Xaa is Lys, Arg, Asp, or Glu.
<220>
   <221> VARIANT
   <222> (7)..(8)
   <223> wherein Xaa is any amino acid.
<220>
   <221> VARIANT
   <222> (9)..(12)
   <223> wherein Xaa is Ala, Val, Leu, Ile, Met, Phe, or Pro.
<220>
   <221> VARIANT
   <222> (13)
   <223> wherein Xaa is any amino acid.
<220>
   <221> VARIANT
   <222> (14)
   <223> wherein Xaa is Ala, val, Leu, Ile, Met, Phe, or Pro.
<220>
   <221> VARIANT
   <222> (15)..(16)
   <223> Wherein Xaa is any amino acid.
<220>
   <221> VARIANT
   <222> (17)..(18)
   <223> wherein Xaa is Ala, val, Leu, Ile, Met, Phe, or Pro.
<220>
   <221> VARIANT
   <222> (19)
   <223> wherein xaa is any amino acid.
<220>
   <221> VARIANT
   <222> (20)..(23)
   <223> Wherein Xaa is Ala, val, Leu, Ile, Met, Phe, or Pro.
<220>
   <221> VARIANT
   <222> (24)..(25)
   <223> wherein Xaa is any amino acid.
<400> 47
   Xaa Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Xaa Xaa Xaa Xaa
               20                  25
<210> 48
   <211> 23
   <212> PRT
   <213> Artificial sequence
<220>
   <223> Description of Artificial sequence: Penetrating Protein consensus Sequence
<220>
   <221> VARIANT
   <222> (1)
   <223> wherein Xaa is Ala, val, Leu, Ile, Met, Phe, or Pro.
<220>
   <221> VARIANT
   <222> (2)
   <223> wherein Xaa is Lys, Arg, Asp, or Glu.
<220>
   <221> VARIANT
   <222> (3)
   <223> Wherein Xaa is any amino acid.
<220>
   <221> VARIANT
   <222> (4)
   <223> wherein Xaa is Ala, val, Leu, Ile, Met, Phe, or Pro.
<220>
   <221> VARIANT
   <222> (5)
   <223> wherein Xaa is any amino acid.
<220>
   <221> VARIANT
   <222> (6)
   <223> wherein Xaa is Lys. Arg, Asp, or Glu.
<220>
   <221> VARIANT
   <222> (7)..(8)
   <223> wherein xaa is any amino acid.
<220>
   <221> VARIANT
   <222> (9)..(12)
   <223> wherein Xaa is Ala, val, Leu, Ile, Met, Phe, or Pro.
<220>
   <221> VARIANT
   <222> (13)
   <223> wherein Xaa is any amino acid.
<220>
   <221> VARIANT
   <222> (14)
   <223> wherein Xaa is Ala, val, Leu, Ile, Met, Phe, or Pro.
<220>
   <221> VARIANT
   <222> (15)..(16)
   <223> wherein Xaa is any amino acid.
<220>
   <221> VARIANT
   <222> (17)..(18)
   <223> wherein Xaa is Ala, val, Leu, Ile, Met, Phe, or Pro.
<220>
   <221> VARIANT
   <222> (19)
   <223> wherein Xaa is any amino acid.
<220>
   <221> VARIANT
   <222> (20)..(23)
   <223> wherein Xaa is Ala, val, Leu, Ile, Met, Phe, or Pro.
<400> 48
   Xaa Xaa Xaa Xaa Xaa Xaa Xaa Xaa Xaa Xaa Xaa Xaa Xaa Xaa Xaa Xaa
    1               5                  10                  15
   Xaa Xaa Xaa Xaa Xaa Xaa Xaa
               20
<210> 49
   <211> 23
   <212> PRT
   <213> Artificial sequence
<220>
   <223> Description of Artificial Sequence: Penetrating Protein Consensus Sequence
<220>
   <221> VARIANT
   <222> (1)..(2)
   <223> wherein xaa is Ala, val, Leu, Ile, Met, Phe, or Pro.
<220>
   <221> VARIANT
   <222> (3)
   <223> wherein xaa is any amino acid.
<220>
   <221> VARIANT
   <222> (4)..(5)
   <223> wherein Xaa is Lys, Arg, Asp, or Glu.
<220>
   <221> VARIANT
   <222> (6)
   <223> wherein Xaa is any amino acid.
<220>
   <221> VARIANT
   <222> (7)..(10)
   <223> wherein Xaa is Ala, val, Leu, Ile, Met, Phe, or Pro.
<220>
   <221> VARIANT
   <222> (11)
   <223> wherein Xaa is any amino acid.
<220>
   <221> VARIANT
   <222> (12)
   <223> wherein Xaa is Ala, val, Leu, Ile, Met, Phe, or Pro.
<220>
   <221> VARIANT
   <222> (13)..(14)
   <223> wherein Xaa is any amino acid.
<220>
   <221> VARIANT
   <222> (15)..(19)
   <223> wherein Xaa is Ala, Val, Leu, Ile, Met, Phe, or Pro.
<220>
   <221> VARIANT
   <222> (20)..(21)
   <223> wherein Xaa is any amino acid.
<220>
   <221> VARIANT
   <222> (22)..(23)
   <223> wherein Xaa is Ala, val, Leu, Ile, Met, Phe, or Pro.
<400> 49
   Xaa Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Xaa Xaa
               20
<210> 50
   <211> 26
   <212> PRT
   <213> Artificial Sequence
<220>
   <223> Description of Artificial Sequence: Penetrating Protein consensus sequence
<220>
   <221> VARIANT
   <222> (1)
   <223> Wherein xaa is Ala, val, Leu, Ile, Met, Phe, Pro or nothing.
<220>
   <221> VARIANT
   <222> (2)
   <223> wherein xaa is any amino acid.
<220>
   <221> VARIANT
   <222> (3)..(4)
   <223> wherein Xaa is any amino acid or nothing.
<220>
   <221> VARIANT
   <222> (5)
   <223> wherein Xaa is Lys, Arg, Asp, or Glu.
<220>
   <221> VARIANT
   <222> (6)
   <223> wherein Xaa is Ala, val, Leu, Ile, Met, Phe, Pro, or nothing.
<220>
   <221> VARIANT
   <222> (7)
   <223> Wherein Xaa is Ala, Val, Leu, Ile, Met, Phe, or Pro.
<220>
   <221> VARIANT
   <222> (8)
   <223> Wherein Xaa is any amino acid or nothing.
<220>
   <221> VARIANT
   <222> (9)..(12)
   <223> Wherein Xaa is Ala, val, Leu, Ile, Met, Phe, or Pro.
<220>
   <221> VARIANT
   <222> (13)
   <223> wherein Xaa is any amino acid.
<220>
   <221> VARIANT
   <222> (14)
   <223> Wherein Xaa is Ala, val, Leu, Ile, Met, Phe, or Pro.
<220>
   <221> VARIANT
   <222> (15)..(16)
   <223> Wherein Xaa is any amino acid.
<220>
   <221> VARIANT
   <222> (17)
   <223> Wherein Xaa is any amino acid or nothing.
<220>
   <221> VARIANT
   <222> (18)
   <223> wherein Xaa is Ala, Val, Leu, Ile, Met, Phe, or Pro.
<220>
   <221> VARIANT
   <222> (19)
   <223> wherein Xaa is Ala, val, Leu, Ile, Met, Phe, Pro, or nothing.
<220>
   <221> VARIANT
   <222> (20)
   <223> wherein Xaa is Lys, Arg, Asp, or Glu.
<220>
   <221> VARIANT
   <222> (21)..(22)
   <223> Wherein Xaa is Ala, Val, Leu, Ile, Met, Phe, or Pro.
<220>
   <221> VARIANT
   <222> (23)..(24)
   <223> wherein Xaa is any amino acid.
<220>
   <221> VARIANT
   <222> (25)..(26)
   <223> wherein Xaa is Ala, val, Leu, Ile, Met, Phe, or Pro.
<400> 50
   Xaa Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Xaa Xaa Xaa Xaa Xaa
               20                  25
<210> 51
   <211> 28
   <212> PRT
   <213> Artificial Sequence
<220>
   <223> Description of Artificial sequence: Penetrating Protein Consensus Sequence
<220>
   <221> VARIANT
   <222> (1)..(2)
   <223> Wherein Xaa is Ala, val, Leu, Ile, Met, Phe, or Pro.
<220>
   <221> VARIANT
   <222> (3)
   <223> wherein Xaa is Lys, Arg, Asp, or Glu.
<220>
   <221> VARIANT
   <222> (4)..(6)
   <223> Wherein Xaa is any amino acid.
<220>
   <221> VARIANT
   <222> (7)
   <223> wherein xaa is Lys, Arg, Asp, or Glu.
<220>
   <221> VARIANT
   <222> (8)
   <223> wherein xaa is Ala, val, Leu, Ile, Met, Phe, or Pro.
<220>
   <221> VARIANT
   <222> (9)
   <223> wherein xaa is Ala, val, Leu, Ile, Met, Phe, Pro, or nothing.
<220>
   <221> VARIANT
   <222> (10)
   <223> wherein Xaa is any amino acid or nothing.
<220>
   <221> VARIANT
   <222> (11)..(14)
   <223> wherein Xaa is Ala, val, Leu, Ile, Met, Phe, or Pro.
<220>
   <221> VARIANT
   <222> (15)
   <223> wherein Xaa is any amino acid.
<220>
   <221> VARIANT
   <222> (16)
   <223> wherein Xaa is Ala, val. Leu, Ile, Met, Phe, or Pro.
<220>
   <221> VARIANT
   <222> (17)..(18)
   <223> Wherein Xaa is any amino acid.
<220>
   <221> VARIANT
   <222> (19)
   <223> wherein Xaa is any amino acid or nothing.
<220>
   <221> VARIANT
   <222> (20)
   <223> wherein Xaa is Ala, val, Leu, Ile, Met, Phe, or Pro.
<220>
   <221> VARIANT
   <222> (21)
   <223> Wherein Xaa is Ala, Val, Leu, Ile, Met, Phe, Pro, or nothing.
<220>
   <221> VARIANT
   <222> (22)
   <223> wherein Xaa is Lys, Arg, Asp, or Glu.
<220>
   <221> VARIANT
   <222> (23).. (24)
   <223> wherein Xaa is Ala, val, Leu, Ile, Met, Phe, or Pro.
<220>
   <221> VARIANT
   <222> (25)..(26)
   <223> wherein Xaa is any amino acid.
<220>
   <221> VARIANT
   <222> (27)..(28)
   <223> wherein Xaa is Ala, val, Leu, Ile, Met, Phe, or Pro.
<400> 51
   Xaa Xaa Xaa Xaa Xaa Xaa Xaa Xaa Xaa Xaa Xaa Xaa Xaa Xaa Xaa Xaa
   1 5 10 15
   Xaa Xaa Xaa Xaa Xaa Xaa Xaa Xaa Xaa Xaa Xaa Xaa
   20 25
<210> 52
   <211> 23
   <212> PRT
   <213> Artificial sequence
<220>
   <223> Description of Artificial Sequence: Penetrating Protein Consensus sequence
<220>
   <221> VARIANT
   <222> (1)..(3)
   <223> wherein Xaa is any amino acid.
<220>
   <221> VARIANT
   <222> (4)
   <223> wherein Xaa is Lys, Arg, Asp, or Glu.
<220>
   <221> VARIANT
   <222> (5)..(10)
   <223> wherein Xaa is Ala, Val, Leu, Ile, Met, Phe, or Pro.
<220>
   <221> VARIANT
   <222> (11)
   <223> Wherein Xaa is any amino acid.
<220>
   <221> VARIANT
   <222> (12)
   <223> Wherein Xaa is Ala, val, Leu, Ile, Met, Phe, or Pro.
<220>
   <221> VARIANT
   <222> (13)..(15)
   <223> wherein Xaa is any amino acid.
<220>
   <221> VARIANT
   <222> (16)
   <223> wherein Xaa is Ala, val, Leu, Ile, Met, Phe, or Pro.
<220>
   <221> VARIANT
   <222> (17)
   <223> wherin Xaa is Lys, Arg, Asp, or Glu.
<220>
   <221> VARIANT
   <222> (18)..(19)
   <223> wherein Xaa is Ala, Val, Leu, Ile, Met, Phe, or Pro.
<220>
   <221> VARIANT
   <222> (20)..(21)
   <223> wherein Xaa is any amino acid.
<220>
   <221> VARIANT
   <222> (22)..(23)
   <223> wherein Xaa is Ala, val, Leu, Ile, Met, Phe, or Pro.
<400> 52
   Xaa Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Xaa Xaa
               20
<210> 53
   <211> 205
   <212> PRT
   <213> Haemophilus influenzae
<400> 53
   Met Lys Asn Tyr His Asp Ile Val Leu Ala Leu Ala Gly Val Cys Gln
   1               5                   10                  15
   Ser Ala Lys Leu Val His Gln Leu Ala Thr Glu Ser Arg Ala Asp Ser
               20                  25                  30
   Glu Thr Phe Leu Thr Ala Leu Asn Ser Leu Phe Ile Thr Gln Pro Gln
           35                  40                  45
   Arg Ile Glu Asp Val Phe Gly Gly Glu Val Arg His Leu Lys Leu Gly
       50                  55                  60
   Leu Glu Thr Leu Ile His Gln Leu Asn Ala Gln Gly Asp Gln Asn Leu
   65                  70                  75                      80
   Thr Arg Tyr Trp Leu Ser Leu Leu Ala Leu Glu Gly Lys Leu Ser Lys
                   85                  90                  95
   Asn Ser Asp Ala Lys Gln Thr Leu Gly Asn Arg Ile Ser Arg Leu Lys
               100                 105                 110
   Glu Gln Glu Ile His Tyr Ala Arg Asp Ser Glu Thr Met Leu Ser Ile
           115                 120                 125
   Met Ala Asn Ile Tyr Ser Asp Ile Ile Ser Pro Leu Gly Lys Lys Ile
       130                 135                 140
   His Ile Leu Gly Ser Pro Asp Tyr Leu Arg Gln Glu Leu Val Gln Asn
   145                 150                 155                     160
   Lys Ile Arg Ala Val Leu Leu Ala Gly Ile Arg Ser Ala Val Leu Trp
                   165                 170                 175
   Lys Gln Met Gly Gly Thr Lys Trp Gln Ile Leu Phe Phe Arg Arg Lys
   180 185 190
   Leu Leu Ala Thr Ala Lys Gln Ile Tyr Ser Ser Ile Tyr
   195 200 205
<210> 54
   <211> 203
   <212> PRT
   <213> Pasturella multocida
<400> 54
   Met Ala Asn Tyr Tyr Asp Ile Thr Leu Ala Leu Ala Gly Val Cys Gln
   1 5 10 15
   Ala Ala Lys Leu Val Gln Gln Phe Ala His Glu Gly Gln Ala Asp Gln
   20 25 30
   Ala Ala Phe Glu Thr Ser Leu Asn Thr Leu Leu Gln Ile Tyr Pro Glu
   35 40 45
   Asp Thr Leu Ala Val Phe Gly Gly Lys Ala Gln Asn Leu Lys Leu Gly
   50 55 60
   Leu Glu Thr Leu Leu Glu Gln Met His Gly Thr Gly Ser Asp Leu Ser
   65 70 75 80
   Arg Tyr Trp Ile Ser Leu Leu Ala Leu Glu Ser Lys Leu Asn Lys Asp
   85 90 95
   Pro His Ala Lys Ala Glu Leu Ala Arg Arg Ile Gln Tyr Leu Pro Thr
   100 105 110
   Gln Leu Glu His Tyr Asp Leu Leu Asp Glu Gln Met Leu Ser Thr Leu
   115 120 125
   Ala Ser Ile Tyr Val Asp Val Ile Ser Pro Leu Gly Lys Lys Ile Gln
   130 135 140
   Val Thr Gly Ser Thr Leu Tyr Leu Gln Gln Leu Ala Met His His Arg
   145 150 155 160
   Ile Arg Ala Cys Leu Leu Ala Gly Ile Arg Ser Ala Val Leu Trp Arg
   165 170 175
   Gln Val Gly Gly Thr Lys Trp Gln Val Leu Phe Ser Arg Arg Lys Ile
   180 185 190
   Ile Ala Met Ala Lys Gln Ile Tyr Ser Ser Leu
   195 200
<210> 55
   <211> 213
   <212> PRT
   <213> Escheria coli
<400> 55
   Met Ala Lys Asn Tyr Tyr Asp Ile Thr Leu Ala Leu Ala Gly Ile Cys
   1               5                   10                  15
   Gln Ser Ala Arg Leu Val Gln Gln Leu Ala His Gln Gly His Cys Asp
               20                  25                  30
   Ala Asp Ala Leu His Val Ser Leu Asn Ser Ile Ile Asp Met Asn Pro
           35                  40                  45
   Ser Ser Thr Leu Ala Val Phe Gly Gly Ser Glu Ala Asn Leu Arg Val
       50                  55                  60
   Gly Leu Glu Thr Leu Leu Gly Val Leu Asn Ala Ser Ser Arg Gln Gly
   65                  70                  75                  80
   Leu Asn Ala Glu Leu Thr Arg Tyr Thr Leu Ser Leu Met Val Leu Glu
                   85                  90                  95
   Arg Lys Leu Ser Ser Ala Lys Gly Ala Leu Asp Thr Leu Gly Asn Arg
               100                 105                 110
   Ile Asn Gly Leu Gln Arg Gln Leu Glu His Phe Asp Leu Gln Ser Glu
           115                 120                 125
   Thr Leu Met Ser Ala Met Ala Ala Ile Tyr Val Asp Val Ile Ser Pro
       130                 135                 140
   Leu Gly Pro Arg Ile Gln Val Thr Gly Ser Pro Ala Val Leu Gln Ser
   145                 150                 155                 160
   Pro Gln Val Gln Ala Lys Val Arg Ala Thr Leu Leu Ala Gly Ile Arg
                   165                 170                 175
   Ala Ala Val Leu Trp His Gln Val Gly Gly Gly Arg Leu Gln Leu Met
               180                 185                 190
   Phe Ser Arg Asn Arg Leu Thr Thr Gln Ala Lys Gln Ile Leu Ala His
           195                 200                 205
   Leu Thr Pro Glu Leu
       210
<210> 56
   <211> 204
   <212> PRT
   <213> vibrio cholerae
<400> 56
   Met Ala Asn Ala Ile Tyr Asp Arg Thr Ile Ala Phe Ala Gly Ile Cys
   1               5                   10                  15
   Gln Ala Val Ala Leu Val Gln Gln Val Ala Lys Asn Gly Tyr Cys Asp
               20                  25                  30
   Ser Asp Ala Phe Glu Thr Ser Leu Lys Ala Ile Thr Cys Thr Asn Pro
           35                  40                  45
   Ser Asn Thr Leu Glu Val Phe Gly His Glu Ser Gln Leu Lys Leu Gly
       50                  55                  60
   Leu Glu Cys Leu Val Lys Gly Ile Asp Ser Thr Pro Ser Gly Ser Glu
   65                  70                  75                  80
   Ile Thr Arg Tyr Leu Ile Ser Leu Met Ala Leu Glu Arg Lys Leu Ser
                   85                  90                  95
   Gly Arg Arg Asp Ala Met Ser Gln Leu Gly Asp Arg Ile Gln Met Ile
               100                 105                 110
   Glu Arg Gln Leu Asp His Phe Asp Leu Phe Asp Asp Gln Met Ile Ser
           115                 120                 125
   Asn Leu Ala Ser Ile Tyr Leu Asp Val Ile Ser Pro Ile Gly Pro Arg
       130                 135                 140
   Ile Gln Val Thr Gly Thr Pro Ala Val Leu Gln Gln Thr Ala Asn Gln
   145                 150                 155                 160
   His Lys Val Arg Ala Leu Leu Leu Ser Gly Ile Arg Cys Ala Val Leu
                   165                 170                 175
   Trp Arg Gln Val Gly Gly Arg Arg Arg His Leu Ile Phe Gly Arg Lys
               180                 185                 190
   Lys Met Ile Glu Gln Ala Gln Ile Leu Leu Ala Arg
           195                 200
<210> 57
   <211> 211
   <212> PRT
   <213> Buchnera aphidicola
<400> 57
   Met Lys Lys Ile His Leu Ile Thr Leu Ser Leu Ala Gly Ile Cys Gln
   1 5 10 15
   Ser Ala His Leu Val Gln Gln Leu Ala Tyr Ser Gly Lys Cys Asp Ser
   20 25 30
   Asn Ala Phe Ser Ile Cys Leu Lys Ser Ile Leu Glu Ile Asn Pro Thr
   35 40 45
   Ser Phe Ile Ala Ile Tyr Gly Asn His Glu Lys Asn Leu Ile Ile Gly
   50 55 60
   Leu Glu Ile Leu Leu Ser Thr Leu Thr Phe Ser Ser Phe Ser Tyr Ser
   65 70 75 80
   Tyr Ile Glu Leu Ile Lys Tyr Ile Ser Asn Met Met Ile Ile Glu Lys
   85 90 95
   Lys Leu Lys Lys Ser Arg Thr Ala Ile Tyr Ser Leu Lys Asn Lys Ile
   100 105 110
   Ser Val Ile Ser Ser Glu Tyr Tyr Leu Asn Tyr Asn Ile Lys Asn Leu
   115 120 125
   Thr Arg Lys Leu Gly Glu Leu Tyr Leu Glu Ile Ile Ser Ser Leu Gly
   130 135 140
   Ser Arg Ile Val Ile Lys Gly Ile Lys Asp Phe Leu Gln Asp His Gln
   145 150 155 160
   Ile Gln Glu Lys Ile Arg Cys Leu Leu Phe Ser Gly Ile Arg Ala Ile
   165 170 175
   Val Leu Trp Lys Gln Tyr Gly Gly Asn Gln Leu Gln Leu Ile Tyr Phe
   180 185 190
   Arg Tyr Phe Ile Ile Lys Lys Ala Lys Lys Ile Leu Tyr His Leu Lys
   195 200 205
   Asp Ala Thr
   210
<210> 58
   <211> 206
   <212> PRT
   <213> Pseudomonas aeruginosa
<400> 58
   Met Ser Asp Pro Arg Gln Gln Leu Ile Ala Leu Gly Ala Val Phe Glu
   1 5 10 15
   Ser Ala Ala Leu Val Asp Lys Leu Ala Arg Thr Gly Gln Ile Ser Glu
   20 25 30
   Ala Pro Leu Gly Cys Met Leu Gly Ser Leu Leu Ala Arg Asn Pro Ala
   35 40 45
   Ser Thr Leu Asp Val Tyr Gly Gly Asp Ser Leu Asn Leu Arg Asp Gly
   50 55 60
   Phe Lys Ala Leu Ala Ser Ala Leu Glu Arg Lys Pro Gly Ser Leu Gln
   65 70 75 80
   Arg Glu Pro Leu Arg Tyr Ala Leu Ala Met Leu Thr Leu Glu Arg Gln
   85 90 95
   Leu Asp Lys Arg Gly Asp Met Leu Asp Leu Ile Gly Gln Arg Leu Asp
   100 105 110
   Gln Val Glu Gln Gln Val Gln His Phe Gly Leu Val His Glu Asn Val
   115 120 125
   Ile Ala Ser Phe Ala Ser Ile Tyr Gln Asp Thr Leu Ser Thr Phe Arg
   130 135 140
   Gln Arg Ile Gln Val His Gly Asp Met Arg His Leu Gln Val Ser Ser
   145 150 155 160
   Asn Ala Ala Arg Ile Arg Ala Leu Leu Leu Ala Gly Ile Arg Ser Ala
   165 170 175
   Arg Leu Trp Arg Gln Leu Gly Gly Ser Arg Trp Gln Met Val Phe Ser
   180 185 190
   Arg Arg Arg Leu Leu Asn Glu Leu Tyr Pro Leu Leu Arg Gly
   195 200 205
<210> 59
   <211> 204
   <212> PRT
   <213> Xylella fastidiosa
<220>
   <223> Description of Artificial Sequence: Penetrating Protein Consensus sequence
<400> 59
   Met Asn Ala Leu Ile Asp Asn Arg Val Leu Ala Leu Ala Gly Val Val
   1 5 10 15
   Gln Ala Leu Gln Gln Val Arg Gln Ile Ala Glu Thr Gly Gln Ser Glu
   20 25 30
   Thr Ser Ala Val Arg Thr Ala Ile Asp Ser Val Leu Arg Ile Asp Ala
   35 40 45
   Glu Ser Pro Glu Ala Val Tyr Gly Arg Ile Arg Asp Leu Thr Gln Gly
   50 55 60
   Leu Gln Leu Leu His Asp Tyr Phe Gly Asn Gln Leu Arg Asp Gln Leu
   65 70 75 80
   Leu Pro Arg Leu Ala Leu Ala Val Leu Gln Leu Glu Arg Arg Phe Ile
   85 90 95
   Arg Asp Thr Ser Ile Val Ala Ala Val Ser Ala Gly Ile Thr Gln Ala
   100 105 110
   Ala His Gln Val Glu Gln Thr Gly Asp Ser Ala His Pro Glu Val Leu
   115 120 125
   Ser Thr Leu Gly Ala Leu Tyr Ala Asn Thr Ile Ser His Leu Arg Pro
   130 135 140
   Arg Ile Ile Val Gln Gly Asn Pro His Tyr Leu Gly Gln Ala Gly Val
   145 150 155 160
   Val Ala Glu Ile Arg Ala Met Leu Leu Ala Ala Leu Arg Ser Ala Val
   165 170 175
   Leu Trp Arg Gln Leu Asn Gly Asn Leu Leu Asp Phe Met Leu Ala Lys
   180 185 190
   Arg Ala Met Ala Ala Ala Thr Glu Arg Ala Leu Arg
   195 200